(19) Europäisches Patentamt
European Patent Office
Office européen des brevets

(11) **EP 4 349 377 B1**

(12) **EUROPEAN PATENT SPECIFICATION**

(45) Date of publication and mention of the grant of the patent:
**18.03.2026 Bulletin 2026/12**

(21) Application number: **22833142.7**

(22) Date of filing: **28.06.2022**

(51) International Patent Classification (IPC):
***A61L 31/04** (2006.01)* ***A61L 29/04** (2006.01)*
***A61L 29/06** (2006.01)* ***A61L 29/08** (2006.01)*
***A61L 31/06** (2006.01)* ***A61L 31/10** (2006.01)*

(52) Cooperative Patent Classification (CPC):
(C-Sets available)
**A61L 29/06; A61L 29/085; A61L 31/06; A61L 31/10**
(Cont.)

(86) International application number:
**PCT/JP2022/025686**

(87) International publication number:
**WO 2023/276995 (05.01.2023 Gazette 2023/01)**

(54) **MEDICAL INSTRUMENT AND METHOD FOR MANUFACTURING SAME**
MEDIZINISCHES INSTRUMENT UND VERFAHREN ZUR HERSTELLUNG DAVON
INSTRUMENT MÉDICAL ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2021 JP 2021107517**

(43) Date of publication of application:
**10.04.2024 Bulletin 2024/15**

(73) Proprietor: **TERUMO Kabushiki Kaisha**
**Tokyo 151-0072 (JP)**

(72) Inventor: **MATSUHASHI, Takeshi**
**Fujinomiya-shi, Shizuoka 418-0015 (JP)**

(74) Representative: **Casalonga**
**Casalonga & Partners**
**Bayerstraße 71/73**
**80335 München (DE)**

(56) References cited:
**WO-A1-2015/029625** **WO-A1-2015/171483**
**WO-A1-2021/054359** **JP-A- 2006 141 555**
**JP-A- 2013 166 838** **JP-A- 2017 519 854**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 349 377 B1

(52) Cooperative Patent Classification (CPC): (Cont.)

C-Sets
**A61L 29/06, C08L 75/04;**
**A61L 29/085, C08L 75/04;**
**A61L 29/085, C08L 101/02;**
**A61L 31/06, C08L 75/04;**
**A61L 31/10, C08L 75/04;**
**A61L 31/10, C08L 101/02**

## Description

### Technical Field

The present invention relates to a medical device and a method for producing the medical device.

### Background Art

A medical device such as a catheter, a guide wire, and a stylet to be inserted into a lumen in a living body such as an airway, a trachea, a digestive tract, a urethra, and a blood vessel, or a tissue is required to have operability by which the tissue is not damaged and the medical device can be reliably inserted into a target site, and the medical device is further required to have an excellent lubricating property in order to avoid damage to a mucous membrane or occurrence of inflammation due to friction during indwelling in the tissue.

In order to satisfy such requirements, it is known that a coating layer containing a maleic acid-based polymer substance in which a ratio of a carboxylate is adjusted to a predetermined range is formed on a surface of a base material of a medical device to be inserted into a living body (for example, WO 2015/029625).

### Summary of Invention

According to the technique disclosed in WO 2015/029625, the medical device can exhibit an excellent lubricating property even under severe conditions. On the other hand, in recent years, with the miniaturization and reduction in diameters of a medical device, an approach of treating a lesion site through a very complicated lumen in a living body, such as delivery of a medical device to a lesion site and treatment for the lesion site through a portion of a lumen in a living body having a small inner diameter, a portion of a lumen in a living body where a plurality of bent portions are continuously present, or the like, is expanding. In addition, with the complexity in medical procedures, the operation of the medical device may take a longer time. Accordingly, in order to maintain the operability of the medical device for a longer period of time even in a case where a lesion site is treated through a very complicated lumen in a living body, there is a demand for a technique of further enhancing a lubrication retaining property (durability) in a surface of the medical device than the related art. More specifically, in a severe situation (for example, in a case where a medical device is delivered to a lesion site through a portion of a lumen in a living body having a small bending radius, a portion of a lumen in a living body where bent portions are continuously present, or a portion of a lumen in a living body having a small inner diameter), the medical device is required to have flexibility capable of following a lumen in a living body or the like in order to deliver the medical device to a desired site. In addition, there is a demand for a medical device that can maintain a high lubricating property even when sliding on the surface of the medical device is repeated and that has excellent sliding durability.

The present invention has been made in view of the above circumstances, and an object of the present invention is to provide a technique by which flexibility (particularly, flexibility of a distal end) and a lubrication retaining property (sliding durability) even under severe situations can be exhibited.

The scope of protection is defined by the claims.

As a result of intensive studies to solve the above problems, the present inventors have found that the above problems can be solved by combining a polymer having a carboxy group or a salt or an ester thereof with a polyurethane having a specific elongation percentage and a specific Young's modulus, and have completed the present invention.

That is, the above object can be achieved by a medical device including a base layer and a surface lubricating layer formed on at least a part of the base layer, in which the surface lubricating layer contains a polymer having a carboxy group, or a salt or an ester thereof, and a polyurethane, and the polyurethane has an elongation percentage of 700% or more and a Young's modulus of 5.0 $N/mm^2$ or more.

### Brief Description of Drawings

[FIG. 1] FIG. 1 is a schematic diagram of a lubrication retaining property evaluation test device (friction tester). In FIG. 1, the reference numeral 1 indicates water; the reference numeral 2 indicates a petri dish; the reference numeral 3 indicates a sample (medical device); the reference numeral 4 indicates a terminal; the reference numeral 5 indicates a load; the reference numeral 6 indicates a moving table; and the reference numeral 10 indicates a friction tester.

[FIG. 2] FIG. 2 is a diagram showing a method for evaluating the presence of a hydrophilic polymer and a polyurethane in a surface lubricating layer and a mixing ratio thereof.

[FIG. 3] FIG. 3 is a cross-sectional view showing a shape of a distal end of a Ni-Ti wire used in Example 16 and Comparative Examples 10 and 11. In FIG. 3, the reference numeral 30 indicates a Ni-Ti wire; the reference numeral 31

indicates a distal end; the reference numeral 32 indicates a distal end straight part; the reference numeral 33 indicates a tapered part; and the reference numeral 34 indicates a rear end portion.

[FIG. 4] FIG. 4 is a schematic diagram of a micro-load meter used in distal end abutting resistance evaluation. In FIG. 4, the reference numeral 40 indicates a micro-load meter; the reference numeral 42 indicates a load cell; the reference numeral 43 indicates a sample; the reference numeral 44 indicates a sample chuck portion; and the reference numeral 46 indicates a pedestal.

[FIG. 5] FIG. 5 is a diagram showing guide wire distal end reachability evaluation using a blood vessel model. A of FIG. 5 is a plan view showing a part of the blood vessel model. In FIG. 5, the reference numeral 50 indicates a blood vessel model; the reference numeral 51 indicates a main pipe; the reference numeral 52 indicates a side branch; the reference numeral 53 indicates a curved portion; and the reference numeral 54 indicates a linear portion. B of FIG. 5 is a diagram showing a shape of a distal end of a guide wire used in the guide wire distal end reachability evaluation using the blood vessel model.

[FIG. 6] FIG. 6 is a diagram showing a test system used in a microparticle test. In FIG. 6, the reference numeral 60 indicates a test system; the reference numeral 61 indicates a hub; the reference numeral 62 indicates a catheter tube; and the reference numeral 63 indicates a mold.

[FIG. 7] FIG. 7 is a partial cross-sectional view schematically showing a lamination configuration on a surface in a representative embodiment of a medical device according to the present invention. In FIG. 7, the reference numeral 100 indicates a medical device; the reference numeral 101 indicates a base layer; and the reference numeral 102 indicates a surface lubricating layer.

[FIG. 8] FIG. 8 is a partial cross-sectional view schematically showing a configuration example having a different lamination configuration on a surface as an application example of the embodiment in FIG. 7. In FIG. 8, the reference numeral 100 indicates a medical device; the reference numeral 101a indicates a base layer core portion; the reference numeral 101b indicates a base material surface layer; and the reference numeral 102 indicates a surface lubricating layer.

## Description of Embodiments

Hereinafter, embodiments of the present invention will be described in detail. The present invention is not limited to the following embodiments, and various modifications can be made within the scope of the claims. It should be understood that an expression of a singular form in the entirety of the present specification includes a concept of a plural form thereof unless otherwise specified. Therefore, it should be understood that an article for the singular form (for example, "a", "an", "the", or the like in the case of English) also includes a concept of the plural form thereof unless otherwise specified. In addition, it should be understood that the terms used in the present specification are used in the meaning generally used in the field unless otherwise specified. Therefore, unless otherwise defined, all technical terms and scientific technical terms used in the present specification have the same meanings as those generally understood by those skilled in the art in the field to which the present invention belongs. When there is a contradiction, the present specification (including definition) takes priority.

In the present specification, "X to Y" indicating a range includes X and Y, and means "X or more and Y or less". In the present specification, "A and/or B" means at least one of A and B, and includes both A and B or either A or B. In addition, unless otherwise specified, operations, measurement of physical properties, and the like are performed under conditions of a room temperature (20°C to 25°C) and a relative humidity ranging from 40% RH to 60% RH.

In addition, in the present specification, when a certain structural unit is defined as "derived" from a certain monomer, it means that the structural unit is a divalent structural unit generated by cleavage in one bond of a polymerizable unsaturated double bond of the corresponding monomer.

## <Medical Device>

A medical device according to one aspect of the present invention includes a base layer and a surface lubricating layer formed on at least a part of the base layer. The surface lubricating layer contains a polymer having a carboxy group, or a salt or an ester thereof, and a polyurethane. The polyurethane has an elongation percentage of 700% or more and a Young's modulus of 5.0 N/mm$^2$ or more. The present invention can provide a medical device that can exhibit flexibility (particularly, flexibility of a distal end) and a lubrication retaining property (sliding durability) under severe situations.

The medical device having the above configuration can exhibit an excellent lubricating property and an excellent lubrication retaining property (sliding durability) even under severe situations (for example, even when the medical device is delivered to a lesion site through a portion of a lumen in a living body having a small bending radius, a portion of a lumen in a living body where bent portions are continuously present, or a portion of a lumen in a living body having a small inner diameter). In addition, in the medical device having the above configuration, the surface lubricating layer has high flexibility, and the flexibility of the medical device can be maintained. Therefore, when the medical device according to the present

invention is a catheter, an operator can cause a distal end of the medical device to easily follow a complicated lesion site and to reach a periphery of a lumen in a living body. Therefore, the medical device having the configuration can be favorably applied to a complicated lesion site.

In the present specification, the "polymer having a carboxy group or a salt or an ester thereof" is also simply referred to as a "hydrophilic polymer" or a "hydrophilic polymer according to the present invention". In addition, the carboxy group of the hydrophilic polymer, or a salt or an ester thereof is also simply referred to as a "carboxylate or the like" or a "carboxylate or the like according to the present invention".

In the present specification, the "polyurethane having an elongation percentage of 700% or more and a Young's modulus of 5.0 N/mm$^2$ or more" is also simply referred to as a "polyurethane" or a "polyurethane according to the present invention".

In the present specification, the "severe situation" means a situation, in which it is difficult to deliver a medical device to a target site, and means that, for example, a site, which satisfies at least one of conditions that a bending radius is small, a plurality of bent portions are continuously present, an inner diameter is small, and the like, is present in a delivery path (for example, a lumen in a living body) from an insertion portion to a lesion. In addition, "a site, which satisfies at least one of conditions that a bending radius is small, a plurality of bent portions are continuously present, an inner diameter is small, and the like, is present in a delivery path (for example, a lumen in a living body) from an insertion portion to a lesion" is also simply referred to as a "complex lesion site".

Hereinafter, preferable embodiments of the medical device according to the present invention will be described with reference to the accompanying drawings.

FIG. 7 is a partial cross-sectional view schematically showing a lamination structure on a surface in a representative embodiment of the medical device according to the present invention (hereinafter, simply referred to as the "medical device"). FIG. 8 is a partial cross-sectional view schematically showing a configuration example having a different lamination structure on a surface as an application example in the present embodiment. In FIGS. 7 and 8, the reference numeral 101 indicates a base layer; the reference numeral 101a indicates a base layer core portion; the reference numeral 101b indicates a base material surface layer; the reference numeral 102 indicates a surface lubricating layer; and the reference numeral 100 indicates a medical device.

As shown in FIGS. 7 and 8, the medical device 100 according to the present embodiment includes the base layer 101, and the surface lubricating layer 102 which is formed (disposed) in a manner of covering at least a part of a surface of the base layer 101 (an example in which the surface lubricating layer 102 is formed (disposed) on the entire surface (whole surface) of the base layer 101 is shown in the drawings) and which contains a hydrophilic polymer and a polyurethane.

Hereinafter, each configuration of the medical device according to the present embodiment will be described.

[Base Layer (Base Material)]

The base layer used in the present invention may be constituted by any material and can be appropriately selected according to the use. Specifically, examples of the material constituting (forming) the base layer 101 include metal materials, polymer materials, and ceramics. Here, as shown in FIG. 7, the base layer 101 in its entirety (all) may be constituted by or formed from any one of the above materials, or as shown in FIG. 8, the base layer 101 may have a structure in which the base material surface layer 101b is constituted or formed by coating a surface of the base layer core portion 101a constituted by or formed from any one of the above materials, with another one of the above materials through an appropriate method. Examples of the latter case include a base layer in which the base material surface layer 101b is formed by coating a surface of the base layer core portion 101a formed from a resin material and the like, with a metal material through an appropriate method (for example, a method in the related art such as plating, metal deposition, and sputtering); and a base layer in which the base material surface layer 101b is formed by coating a surface of the base layer core portion 101a formed from a hard reinforcing material such as a metal material and a ceramic material, with a polymer material softer than the reinforcing material such as a metal material through an appropriate method (for example, a method in the related art such as dipping, spraying, and coating and printing) or formed by performing complexation (appropriate reaction processing) of a reinforcing material for the base layer core portion 101a and a polymer material for the base material surface layer 101b. Accordingly, the base layer core portion 101a may be a multilayered structure obtained by laminating different materials in a multiple layer, or a structure (for example, a complex) obtained by connecting members formed from different materials for each part of the medical device. In addition, another middle layer (not shown) may be formed between the base layer core portion 101a and the base material surface layer 101b. Further, the base material surface layer 101b may also be a multilayered structure obtained by laminating different materials in a multiple layer, or a structure (for example, a complex) obtained by connecting members formed from different materials for each part of the medical device.

Among the materials constituting or forming the above base layer 101, the metal material is not particularly limited. A metal material, which is generally used in the medical device such as a catheter, a stent, and a guide wire, is used. Specific examples thereof include: various types of stainless steel (SUS) such as SUS304, SUS316, SUS316L,

SUS420J2, and SUS630; gold; platinum; silver; copper; nickel; cobalt; titanium; iron; aluminum; tin; and various types of alloys such as a nickel-titanium (Ni-Ti) alloy, a nickel-cobalt (Ni-Co) alloy, a cobalt-chrome (Co-Cr) alloy, and a zinc-tungsten (Zn-W) alloy. One type thereof may be independently used, or two or more types thereof may be used together. As the above metal material, an optimal metal material may be appropriately selected for the base layer intended to be used for a catheter, a stent, and a guide wire.

In addition, among the materials constituting or forming the above base layer 101, the polymer material is not particularly limited. A polymer material, which is generally used in the medical device such as a catheter, a stent, and a guide wire, is used. Specific examples thereof include: a polyamide resin; polyethylene such as linear low-density polyethylene (LLDPE), low-density polyethylene (LDPE), high-density polyethylene (HDPE), and modified polyethylene; a polyolefin resin such as polypropylene; a polyester resin such as polyethylene terephthalate (PET); a styrene resin such as polystyrene; a cyclic polyolefin resin; a modified polyolefin resin; an epoxy resin; a urethane resin; a diallyl phthalate resin (an allyl resin); a polycarbonate resin; a fluorine resin; an amino resin (for example, a urea resin, a melamine resin, and a benzoguanamine resin); an acrylic resin; a polyacetal resin; a vinyl acetate resin; a phenol resin; a vinyl chloride resin (for example, polyvinyl chloride (PVC)); polytetrafluoroethylene (PTFE); a silicone resin; a polyether resin such as polyether ether ketone (PEEK); and a polyimide resin. Among them, a urethane resin, a vinyl chloride resin (polyvinyl chloride (PVC)), and polyethylene terephthalate (PET) are preferable, a urethane resin and a vinyl chloride resin (polyvinyl chloride (PVC)) are more preferable, and a vinyl chloride resin is particularly preferable. One type of the above polymer materials may be independently used, or two or more types thereof may be used together. As the above polymer material, an optimal polymer material may be appropriately selected for the base layer intended to be used for a catheter, a stent, and a guide wire.

In addition, the shape of the above base layer is not particularly limited. The shape thereof is appropriately selected depending on a usage form, for example, a sheet form, a line form (wire), and a tube form.

[Surface Lubricating Layer]

The surface lubricating layer in the present invention is formed on at least a part of the base layer, and contains a hydrophilic polymer and a polyurethane. Here, the surface lubricating layer does not need to be formed on the entire surface of the base layer. The surface lubricating layer may be formed on (a part of) a surface part of the base layer that comes into contact with an aqueous liquid (for example, a body fluid, blood, or physiological saline). The hydrophilic polymer exhibits a lubricating property at the time of wetting (for example, during contact with an aqueous liquid such as a body fluid, blood, or physiological saline; the same applies hereinafter), and is physically entangled (compatible) with a polyurethane in the surface lubricating layer. The polyurethane according to the present invention has an elongation percentage of 700% or more and a Young's modulus of 5.0 $N/mm^2$ or more. With such characteristics, the polyurethane can follow the expansion of the hydrophilic polymer and can maintain the film structure even if the hydrophilic polymer expands at the time of wetting. In addition, even if the hydrophilic polymer expands at the time of wetting, elution of the hydrophilic polymer can be inhibited or prevented. Therefore, according to the surface lubricating layer in the present invention, the lubrication retaining property (sliding durability) under severe situations can be improved. In addition, the polyurethane has appropriate flexibility. Therefore, the medical device according to the present invention can be delivered to a periphery of a lumen in a living body even if there is a complicated lesion site. In addition, the polyurethane can follow the expansion of the hydrophilic polymer at the time of wetting, and therefore, the surface lubricating layer can ensure a space sufficient for containing an aqueous liquid (for example, a body fluid or blood). Therefore, the hydrophilic polymer can exhibit the lubricating property by coming into contact with a sufficient amount of an aqueous liquid even under high-load conditions.

The above mechanism is presumed and does not limit the technical scope of the present invention.

In the present invention, as long as the effects of the present invention are not affected, another layer may be provided between the surface lubricating layer and the base layer. It is preferable that the surface lubricating layer is positioned directly on the base layer. In addition, as long as the effects of the present invention are not affected, another layer may be provided on the surface lubricating layer. It is preferable that another layer is not disposed on the surface lubricating layer (the surface lubricating layer is the outermost layer). According to the aspect, the effects (for example, the lubrication retaining property and the lubricating property) generated by the present invention can be effectively exhibited.

In addition, a thickness of the surface lubricating layer is not particularly limited. From the viewpoint of the lubricating property, the durability (lubrication retaining property), the flexibility, the adhesion to an adjacent layer (for example, a base layer), and the like, the thickness (dry film thickness) of the surface lubricating layer is preferably 0.01 μm to 20 μm, more preferably 0.1 μm to 15 μm, and still more preferably 1 μm to 10 μm.

(Hydrophilic Polymer)

The surface lubricating layer according to the present invention contains a polymer (hydrophilic polymer) having

a carboxy group, or a salt or an ester thereof (a carboxylate or the like). When the hydrophilic polymer is brought into contact with an aqueous liquid, a carboxylate or the like (for example, an alkali metal salt of a carboxy group) present in the hydrophilic polymer is swollen and gelled in the aqueous liquid to exhibit the lubricating property.

Examples of the hydrophilic polymer include: hyaluronic acid, or alkali metal salts or Group 2 metal salts thereof; polyacrylic acid, or alkali metal salts or Group 2 metal salts thereof; polymers having a structural unit represented by the following formula (1); alginic acid, or alkali metal salts or Group 2 metal salts thereof; carboxymethyl cellulose, or alkali metal salts or Group 2 metal salts thereof; and xanthan gum, or alkali metal salts or Group 2 metal salts thereof. Among them, hyaluronic acid or alkali metal salts or Group 2 metal salts thereof, polyacrylic acid or alkali metal salts or Group 2 metal salts thereof, and a polymer having a structural unit represented by the following formula (1) are preferable, polyacrylic acid or alkali metal salts or Group 2 metal salts thereof, and a polymer having a structural unit represented by the following formula (1) are more preferable, and a polymer having a structural unit represented by the following formula (1) is particularly preferable. These hydrophilic polymers can exhibit a higher lubrication retaining property (sliding durability) under severe situations. In addition, these hydrophilic polymers can exhibit a higher lubricating property during the contact with an aqueous liquid.

[Chem. 1]

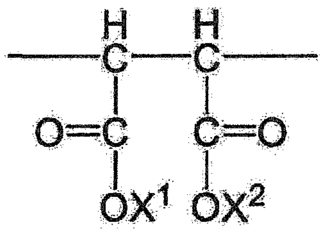

(1)

The surface lubricating layer may contain one type of the above hydrophilic polymers, or may contain two or more types of the hydrophilic polymers.

That is, in a preferable embodiment of the present invention, the polymer (hydrophilic polymer) is at least one type selected from the group consisting of hyaluronic acid or alkali metal salts or Group 2 metal salts thereof, polyacrylic acid or alkali metal salts or Group 2 metal salts thereof, and a polymer having a structural unit represented by the above formula (1). In a more preferable embodiment of the present invention, the polymer (hydrophilic polymer) is at least one selected from the group consisting of polyacrylic acid or alkali metal salts or Group 2 metal salts thereof, and the polymer having a structural unit represented by the above formula (1). In a particularly preferable embodiment of the present invention, the polymer (hydrophilic polymer) is the polymer having a structural unit represented by the above formula (1) .

Examples of the alkali metal salts or Group 2 metal salts of hyaluronic acid include potassium salts, sodium salts, calcium salts, lithium salts, and magnesium salts of hyaluronic acid. The alkali metal salts or Group 2 metal salts of hyaluronic acid may be in the form in which all carboxy groups form a salt or in the form in which some carboxy groups form a salt. The alkali metal salts or Group 2 metal salts of hyaluronic acid preferably have a form in which all carboxy groups form a salt. From the viewpoint of a higher lubrication retaining property (sliding durability) and a higher lubricating property during contact with an aqueous liquid, sodium hyaluronate and potassium hyaluronate are preferable, and sodium hyaluronate is more preferable.

Examples of the alkali metal salts or Group 2 metal salts of polyacrylic acid include potassium salts, sodium salts, calcium salts, and magnesium salts of polyacrylic acid. The alkali metal salts or Group 2 metal salts of polyacrylic acid may be in the form in which all carboxy groups form a salt or in the form in which some carboxy groups form a salt. The alkali metal salts or Group 2 metal salts of polyacrylic acid preferably have a form in which all carboxy groups form a salt. From the viewpoint of a higher lubrication retaining property (sliding durability) and a higher lubricating property during contact with an aqueous liquid, sodium polyacrylate and potassium polyacrylate are preferable, and sodium polyacrylate is more preferable.

An aspect in which the hydrophilic polymer is a polymer having a structural unit represented by the following formula (1) will be described.

[Chem. 2]

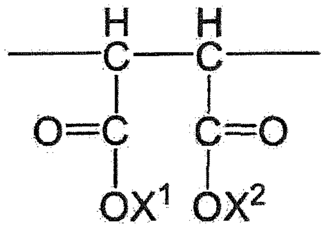

(1)

In the above formula (1), $X^1$ and $X^2$ each independently represent a hydrogen atom, an alkali metal, a Group 2 metal, or a linear or branched alkyl group having 1 to 24 carbon atoms. In this case, $X^1$ and $X^2$ may be the same as or different from each other. Here, examples of the alkali metal include lithium, sodium, potassium, rubidium, and cesium. In addition, examples of the Group 2 metal include magnesium, calcium, strontium, and barium. When $X^1$ or $X^2$ represents a Group 2 metal, adjacent carboxy groups are linked via $X^1$ or $X^2$ (-C(=O)-O-($X^1$ or $X^2$)-O-C(=O)-)). In addition, examples of the linear or branched alkyl group having 1 to 24 carbon atoms include a methyl group, an ethyl group, a propyl group, an isopropyl group, a butyl group, an isobutyl group, a sec-butyl group, a tert-butyl group, a pentyl group, an isopentyl group, a tert-pentyl group, a neopentyl group, a hexyl group, an isohexyl group, a heptyl group, an octyl group, a nonyl group, a decyl group, an undecyl group, a dodecyl group, a 2-ethylhexyl group, a tridecyl group, a tetradecyl group, a pentadecyl group, a hexadecyl group, a heptadecyl group, an octadecyl group, a nonadecyl group, an eicosyl group, a heneicosyl group, and a docosyl group. Among them, from the viewpoint of improving the lubrication retaining property and the lubricating property, $X^1$ and $X^2$ each independently preferably represent sodium, potassium, magnesium, calcium, or a linear or branched alkyl group having 1 to 8 carbon atoms, more preferably represent sodium, calcium, or a linear or branched alkyl group having 1 to 3 carbon atoms, and particularly preferably represent sodium or an ethyl group. From the viewpoint of improving the lubrication retaining property and the lubricating property, it is preferable that at least one of $X^1$ and X2 preferably represents an alkali metal or a linear or branched alkyl group having 1 to 8 carbon atoms, it is more preferable that one of $X^1$ and $X^2$ represents sodium or potassium and the other one represents a linear or branched alkyl group having 1 to 3 carbon atoms, and it is particularly preferable that one of $X^1$ and $X^2$ represents sodium and the other one represents an ethyl group. In particular, when one of $X^1$ and $X^2$ is sodium and the hydrophilic polymer comes into contact with the aqueous liquid, sodium ions are dissociated in the aqueous liquid, and the carboxy group as a hydrophilic group is negatively charged ($-COO^-$). Therefore, a difference in ion concentrations occurs between a periphery of the hydrophilic polymer and the aqueous liquid, and water molecules are easily incorporated into the polymer. At the same time, the hydrophilic groups are in an anionic form, and therefore, Coulomb repulsion between the hydrophilic groups acts to enlarge a network structure formed by the hydrophilic polymer in the aqueous liquid, and a larger number of water molecules can be incorporated (the hydrophilic polymer can be more swollen). Therefore, the hydrophilic polymer can exhibit a particularly excellent lubricating property and lubrication retaining property. The form in which $X^1$ or $X^2$ represents an alkali metal or a Group 2 metal is obtained by subjecting a polymer in which $X^1$ and $X^2$ represent hydrogen atoms to an alkali treatment. Specifically, the alkali treatment can be performed, for example, by adding an alkali such as sodium hydrogen carbonate to a polymer solution in which $X^1$ and $X^2$ represent hydrogen atoms. A coating liquid for forming a lubricating layer may be prepared using the hydrophilic polymer obtained as described above. Alternatively, as described in WO 2015/029625, the alkali treatment may be performed using an alkali such as sodium hydrogen carbonate after the lubricating layer is formed using the polymer in which $X^1$ and $X^2$ represent hydrogen atoms.

The structural unit represented by the above formula (1) may be only one type of structural unit represented by the above formula (1), or two or more types of structural units represented by the above formula (1). The structural unit represented by the above formula (1) is preferably one type of structural unit represented by the above formula (1). In the latter case, the plurality of structural units may be present in a block form or in a random form. For example, as described in WO 2015/029625, a structural unit (1) may be constituted by a structural unit (a) represented by the above formula (1) in which $X^1$ represents a hydrogen atom, an alkali metal, or a Group 2 metal, and $X^2$ represents a linear or branched alkyl group having 1 to 24 carbon atoms, and a structural unit (b) represented by the above formula (1) in which $X^1$ and $X^2$ each independently represent a hydrogen atom, an alkali metal, or a Group 2 metal. In this case, the composition of the structural unit (a):the structural unit (b) may be 100:2 to 100:50 (preferably 100:2 to 100:43) (molar ratio). The composition is substantially the same as a ratio of a charged amount (mol) of a monomer corresponding to each structural unit to a total charged amount (mol) of all monomers in the production of the polymer.

In addition, the polymer having the structural unit represented by the above formula (1) may have a structural unit derived from another monomer (another structural unit) in addition to the structural unit represented by the above formula (1). The other monomer is not particularly limited as long as it does not inhibit the effects generated by the present invention (for example, the lubrication retaining property and lubricating property under severe situations). Specific examples

thereof include: alkyl vinyl ethers; acrylamide or a derivative thereof; vinylpyrrolidone; acrylic acid, methacrylic acid, and derivatives thereof; a diene compound; and a monomer which has sugar and phospholipid in a side chain. More specific examples thereof include methyl vinyl ethers, ethyl vinyl ethers, propyl vinyl ethers, acrylic acid, methacrylic acid, N-methyl acrylamide, N,N-dimethyl acrylamide, acrylamide, acryloyl morpholine, N,N-dimethylaminoethyl acrylate, vinylpyrrolidone, 2-methacryloyloxyethyl phosphorylcholine, 2-methacryloyloxyethyl-D-glycoside, 2-methacryloyloxyethyl-D-mannoside, vinyl methyl ethers, and hydroxyethyl methacrylate. Among them, in consideration of the lubricating property at the time of wetting, the lubricating property and the lubrication retaining property at the time of wetting under severe situations, and the like, alkyl vinyl ethers are preferable, methyl vinyl ethers and ethyl vinyl ethers are more preferable, and methyl vinyl ethers are particularly preferable. The hydrophilic polymer preferably contains substantially no structural unit derived from maleic anhydride having the following structure. Here, "the hydrophilic polymer contains substantially no structural unit derived from maleic anhydride" means that a content of the structural unit derived from maleic anhydride having the following structure is less than 5 mol% (lower limit: 0 mol%) with respect to all structural units. The presence/absence of a structural unit derived from maleic anhydride can be checked by infrared spectroscopy.

[Chem. 3]

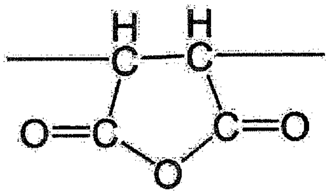

In one embodiment of the present invention, the hydrophilic polymer is a copolymer constituted by the structural unit represented by the above formula (1) and a structural unit derived from an alkyl vinyl ether ($-_2HC-CH(OX^3)-$). Here, in a formula representing a structural unit derived from an alkyl vinyl ether: $-_2HC-CH(OX^3)-$, $X^3$ represents a linear or branched alkyl group having 1 to 24 carbon atoms. Here, "the linear or branched alkyl group having 1 to 24 carbon atoms" has the same definition as that of the alkyl group in the above formula (1), and therefore, the description thereof is omitted here. Among them, $X^3$ preferably represents a linear or branched alkyl group having 1 to 8 carbon atoms, more preferably represents a linear or branched alkyl group having 1 to 3 carbon atoms, still more preferably represents a methyl group or an ethyl group, and particularly preferably represents a methyl group (that is, the other structural unit is represented by the formula: $-_2HC-CH(OCH_3)-$). In the present embodiment, the copolymer may have a structure including the structural unit represented by the above formula (1) and the structural unit derived from an alkyl vinyl ether ($-_2HC-CH(OX^3)-$) as one structural unit as shown in the following structure. Alternatively, each structural unit may be a block form or a random form.

[Chem. 4]

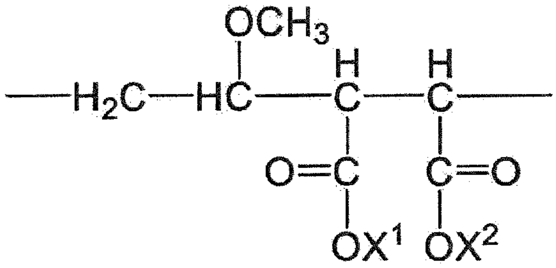

When the hydrophilic polymer is a copolymer constituted by the structural unit represented by the above formula (1) and a structural unit derived from another monomer (another structural unit, particularly a structural unit derived from an alkyl vinyl ether; the same applies hereinafter), a composition of each structural unit (a monomer forming each structural unit) is not particularly limited, and is appropriately selected in consideration of desired effects (for example, the lubrication retaining property and lubricating property under severe situations). Specifically, the hydrophilic polymer is preferably constituted by 20 mol% to 80 mol% of the structural unit represented by the above formula (1) and 80 mol% to 20 mol% of the other structural unit, more preferably constituted by 30 mol% to 70 mol% of the structural unit represented by the above formula (1) and 70 mol% to 30 mol% of the other structural unit, and particularly preferably constituted by 45 mol% to 55 mol% of the structural unit represented by the above formula (1) and 55 mol% to 45 mol% of the other structural unit. Here, a total amount of the structural unit represented by the above formula (1) and the other structural unit is 100 mol%. The composition is substantially the same as a ratio of a charged amount (mol) of a monomer corresponding to each structural unit to a total charged amount (mol) of all monomers in the production of the polymer.

The hydrophilic polymer may have an insolubilizing form as long as the hydrophilic polymer has the degree of freedom in a molecular chain and can take a water-containing form. The insolubilizing form is not particularly limited as long as the hydrophilic polymer has the degree of freedom in a molecular chain and can take a water-containing form. Specific examples thereof include: an amidated compound, an anhydride compound, a halide, an etherified compound, a hydrolyzate, an acetal compound, a formalated compound, an alkylol compound, a quaternary compound, a diazo compound, a hydrazide compound, a sulfonated compound, a nitrogenous compound, and an ion complex which can be obtained through condensation, addition, substitution, oxidation, reduction reaction, and the like of the above hydrophilic polymer; and a crosslinking substance of a substance which has two or more reactive functional groups, and a hydrophilic polymer, the reactive functional group being a diazonium group, an azide group, an isocyanate group, an acid chloride group, an acid anhydride group, an imino carbonate ester group, an amino group, a carboxy group, an epoxy group, a hydroxy group, and an aldehyde group. When such a hydrophilic polymer comes into contact with a body fluid or blood, frictional resistance with respect to a lumen in a living body or tissues can be remarkably reduced, and the hydrophilic polymer can be used as a lubricant. In addition, a derivative which can be obtained through condensation, an addition reaction, a substitution reaction, or the like of the hydrophilic polymers and a substance which is partially crosslinked or the like are similarly effective as a lubricant.

In a preferable aspect of the present invention, the hydrophilic polymer has the following structural unit. In a more preferable aspect of the present invention, the hydrophilic polymer is constituted by the following structural unit.

[Chem. 5]

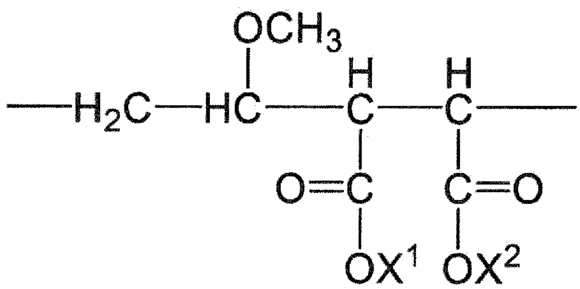

In a particularly preferable aspect of the present invention, the hydrophilic polymer has the following structural unit. In a more preferable aspect of the present invention, the hydrophilic polymer is constituted by the following structural unit.

[Chem. 6]

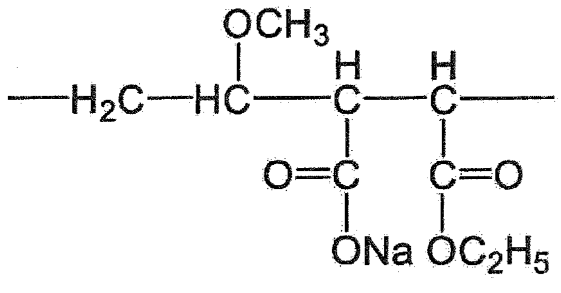

The molecular weight of the hydrophilic polymer is appropriately selected in consideration of desired effects (for example, the lubrication retaining property and the lubricating property under severe situations). Specifically, the weight average molecular weight (Mw) of the hydrophilic polymer is preferably 10000 to 7000000, and more preferably 100000 to 5000000. In the present specification, the molecular weight (weight average molecular weight) of the hydrophilic polymer is measured by gel permeation chromatography (GPC) using polystyrene as a standard substance and tetrahydrofuran (THF) as a mobile phase. The molecular weight of the hydrophilic polymer can also be calculated based on the type of the repeating unit and the number of the repeating units.

(Polyurethane)

The surface lubricating layer according to the present invention contains a polyurethane in addition to the above

polymer (hydrophilic polymer) having a carboxy group or a salt or an ester thereof. The polyurethane herein has an elongation percentage of 700% or more and a Young's modulus of 5.0 N/mm$^2$ or more. The hydrophilic polymer is generally dissolved in water. Therefore, the hydrophilic polymer is gradually eluted at the time of wetting (for example, during the contact with an aqueous liquid such as a body fluid, blood, and physiological saline), and the lubrication retaining property (sliding durability) deteriorates. In contrast, the polyurethane has an appropriate balance between flexibility (an elongation percentage) and hardness (a Young's modulus) and exhibits high compatibility with the hydrophilic polymer. When the polyurethane is combined with the hydrophilic polymer, the hydrophilic polymer is entangled (compatible) with the polyurethane (particularly, a soft segment part) physically (without causing a chemical reaction) in a nano order in the surface lubricating layer. Accordingly, elution of the hydrophilic polymer at the time of wetting (for example, during the contact with the body fluid or blood) can be prevented. In addition, the polyurethane has a specific elongation percentage (flexibility) as described above. Therefore, even when the hydrophilic polymer absorbs a large amount of the aqueous liquid and swells at the time of wetting, the polyurethane has elasticity enough to withstand the expansion and can favorably follow the expansion (swelling) of the hydrophilic polymer. Therefore, when the medical device comes into contact with a wall of a lumen in a living body such as a blood vessel, a sufficient amount of the aqueous liquid seeps out to a surface of the surface lubricating layer, and thus, the surface lubricating layer prevents the medical device from coming into contact with the wall of a lumen in a living body. Therefore, the surface lubricating layer can exhibit an excellent lubricating property even under severe situations. In addition, the polyurethane has flexibility, and therefore, the flexibility of the medical device itself including the surface lubricating layer can be maintained. When the medical device including the surface lubricating layer according to the present invention is a catheter or a guide wire, the flexibility of a distal end of the catheter or the guide wire can be maintained (a distal end abutting resistance is low). Therefore, when an operator operates the medical device (for example, a catheter or a guide wire) according to the present invention, the medical device can easily pass through a portion of a lumen in a living body where the bending is strong (the bending radius is small) or a plurality of bent portions are continuously present, and the distal end of the catheter or the guide wire can reach a periphery of the lumen in a living body. In addition, the surface lubricating layer contains the polyurethane having a specific Young's modulus (hardness) as described above, and therefore, the surface lubricating layer (particularly, in a vicinity of an interface with the base layer) has a film strength equal to or larger than a certain value. Therefore, the surface lubricating layer is less likely to be peeled off from the base layer under repeated sliding abrasion (for example, a site where a plurality of bent portions are continuously present) or when passing through a narrow site, and the film structure can be maintained. Therefore, the surface lubricating layer according to the present invention can improve the lubrication retaining property (sliding durability) under severe situations. The above mechanism is presumed and does not limit the technical scope of the present invention.

The polyurethane has an elongation percentage of 700% or more and a Young's modulus of 5.0 N/mm$^2$ or more. Here, when the elongation percentage of the polyurethane is less than 700%, it is not possible to appropriately cope with the absorption and swelling of the aqueous liquid by the hydrophilic polymer, and the lubricating property deteriorates. In addition, when the Young's modulus of the polyurethane is less than 5.0 N/mm$^2$, the film strength becomes weak, and the film structure cannot be maintained under the repeated sliding abrasion (for example, a site where a plurality of bent portions are continuously present) or when passing through a narrow site. Therefore, the lubrication retaining property (sliding durability) deteriorates. Therefore, the hydrophilic polymer is eluted at the time of wetting. In addition, when the hydrophilic polymer absorbs a large amount of the aqueous liquid and swells at the time of wetting, the hydrophilic polymer cannot follow the expansion (swelling), and the film structure of the surface lubricating layer is broken. Therefore, the surface lubricating layer formed by using such a polyurethane is inferior in the lubrication retaining property (sliding durability) under severe situations. From the viewpoint of further improving the lubrication retaining property (sliding durability) and flexibility under severe situations, the elongation percentage of the polyurethane is preferably more than 700%, more preferably more than 1000%, still more preferably 1100% or more, and particularly preferably more than 1100%. The upper limit of the elongation percentage of the polyurethane is, for example, 3000% or less, and preferably 2000% or less. In addition, from the viewpoint of further improving the lubrication retaining property (sliding durability) under severe situations and the film strength, the Young's modulus of the polyurethane is preferably more than 5.0 N/mm$^2$, more preferably more than 5.2 N/mm$^2$, and particularly preferably 5.4 N/mm$^2$ or more. The upper limit of the Young's modulus of the polyurethane is, for example, 800 N/mm$^2$ or less, preferably 320 N/mm$^2$ or less, more preferably less than 300 N/mm$^2$, and particularly preferably less than 50 N/mm$^2$.

That is, in a preferable embodiment of the present invention, the polyurethane has an elongation percentage of more than 700% and a Young's modulus of more than 5.0 N/mm$^2$. In a more preferable embodiment of the present invention, the polyurethane has an elongation percentage of more than 1000% and a Young's modulus of more than 5.2 N/mm$^2$. In a further preferable embodiment of the present invention, the polyurethane has an elongation percentage of 1100% or more and a Young's modulus of 5.4 N/mm$^2$ or more. In a particularly preferable embodiment of the present invention, the polyurethane has an elongation percentage of more than 1100% and a Young's modulus of 5.4 N/mm$^2$ or more.

Regarding the surface lubricating layer, when the hydrophilic polymer absorbs a large amount of the aqueous

liquid at the time of wetting and swells, the polyurethane also stretches or contracts, and deforms together. Therefore, the polyurethane in the surface lubricating layer is preferably a material that deforms by swelling of the hydrophilic polymer when the hydrophilic polymer absorbs a large amount of the aqueous liquid at the time of wetting and swells. That is, the polyurethane preferably has a small stress when receiving an external force. From such a viewpoint, the 100% modulus of the polyurethane is preferably less than 6.9 $N/mm^2$, more preferably 6.2 $N/mm^2$ or less, still more preferably less than 6.2 $N/mm^2$, yet still more preferably 3.0 $N/mm^2$ or less, and particularly preferably less than 2.5 $N/mm^2$. The lower limit of the 100% modulus of the polyurethane is, for example, 0.5 $N/mm^2$ or more, and preferably 0.7 $N/mm^2$ or more. When the polyurethane has a 100% modulus in such a range, the polyurethane deforms together and can favorably follow expansion (swelling) of the hydrophilic polymer even if the hydrophilic polymer absorbs a large amount of the aqueous liquid and swells at the time of wetting. Therefore, the surface lubricating layer can exhibit an excellent lubrication retaining property (durability) and flexibility even under severe situations.

That is, in a preferable embodiment of the present invention, the polyurethane has an elongation percentage of more than 700%, a Young's modulus of more than 5.0 $N/mm^2$, and a 100% modulus of less than 6.9 $N/mm^2$. In a more preferable embodiment of the present invention, the polyurethane has an elongation percentage of more than 1000%, a Young's modulus of more than 5.2 $N/mm^2$, and a 100% modulus of 6.2 $N/mm^2$ or less. In a still more preferable embodiment of the present invention, the polyurethane has an elongation percentage of more than 1000%, a Young's modulus of more than 5.2 $N/mm^2$, and a 100% modulus of less than 6.2 $N/mm^2$. In a yet still more preferable embodiment of the present invention, the polyurethane has an elongation percentage of 1100% or more, a Young's modulus of 5.4 $N/mm^2$ or more, and a 100% modulus of 3.0 $N/mm^2$ or less. In a particularly preferable embodiment of the present invention, the polyurethane has an elongation percentage of more than 1100%, a Young's modulus of 5.4 $N/mm^2$ or more, and a 100% modulus of less than 2.5 $N/mm^2$.

In the present specification, values measured according to the following method are adopted as the elongation percentage, Young's modulus, and 100% modulus of the polyurethane.

(Measurement of Elongation Percentage (Elongation), Young's Modulus, and 100% Modulus)

A liquid in which polyurethane is dissolved or dispersed is charged into a Teflon (registered trademark) coated petri dish so as to form a film thickness of 500 μm, followed by drying at room temperature (25°C) for 15 hours, at 80°C for 6 hours, and at 120°C for 20 minutes, and a film is formed. The obtained film is cut into a dumbbell-shaped test piece (No. 3) to prepare a sample. The sample is measured for the elongation percentage (elongation) (%), Young's modulus ($N/mm^2$), and 100% modulus ($N/mm^2$) at a tensile speed of 200 mm/min using a Tensilon universal testing machine (manufactured by ORIENTEC CO., LTD.) according to JIS K6251: 2017 (old JIS K6301). The elongation percentage is a value obtained by dividing the elongation at the breakage of the test piece by an original length of the test piece to obtain a ratio and representing the ratio in percentage. The Young's modulus indicates a relation between a stress and a strain in an elastic region (initial proportional region) of the material, and is a value obtained by dividing the tensile stress at pulling of the test piece by the amount of change (strain) with respect to the original length of the test piece. The 100% modulus is a tensile stress when the elongation percentage of the test piece is 100%.

The polyurethane is a reaction product of an isocyanate compound having an isocyanate group (NCO group) and a polyhydric alcohol having two or more hydroxy groups (OH groups) in one molecule, and has a soft segment and a hard segment. Examples of the polyurethane include an ester-ether-based polyurethane, an ether-based polyurethane, an ester-based polyurethane, a carbonate-based polyurethane, and an acrylic polyurethane. One type of the polyurethane may be independently used, or two types or more thereof may be used together.

The above isocyanate compound forming the polyurethane is an organic compound having two or more isocyanate groups in one molecule (polyvalent isocyanate compound). Examples of the isocyanate compound include an aliphatic isocyanate, an alicyclic isocyanate, and an aromatic isocyanate.

Specific examples of the aliphatic isocyanate compound include 1,6-hexamethylene diisocyanate (HDI).

Specific examples of the alicyclic isocyanate compound include dicyclohexylmethane diisocyanate, isophorone diisocyanate (IPDI), 1,3-cyclohexane diisocyanate, and 1,4-cyclohexane diisocyanate.

Examples of the aromatic isocyanate compound include 2,4-tolylene diisocyanate, 2,6-tolylene diisocyanate, 4,4'-diphenylmethane diisocyanate, 2,4'-diphenylmethane diisocyanate, 2,2'-diphenylmethane diisocyanate, xylylene diisocyanate (XDI), and naphthylene diisocyanate (NDI).

Only one type of these isocyanate compounds may be independently used, or two or more types thereof may be used together.

Among the isocyanate compounds, an aliphatic isocyanate compound and an alicyclic isocyanate compound are preferable, and 1,6-hexamethylene diisocyanate (HDI) and isophorone diisocyanate (IPDI) are more preferable.

A content of the structural unit derived from the isocyanate compound is preferably more than 3 mol% and less than 25 mol%, and more preferably 5 mol% to 18 mol% based on all structural units of the polyurethane.

Specific examples of the polyhydric alcohol include: low-molecular-weight diols such as ethylene glycol,

propylene glycol, 1,3-propanediol, 1,4-butanediol, 1,5-pentanediol, 3-methyl-1,5-pentanediol, 1,6-hexanediol, neopentyl glycol, diethylene glycol, trimethylene glycol, triethylene glycol, tetraethylene glycol, dipropylene glycol, tripropylene glycol, hexanediol, and cyclohexanedimethanol; polyether diols obtained by addition polymerization of at least one of low-molecular-weight compounds having three or more hydroxy groups such as glycerin, trimethylolpropane (TMP), and pentaerythritol and the above low-molecular-weight diols with ethylene oxide, propylene oxide, tetrahydrofuran, or the like; a compound having a hydroxy group and a carboxy group in one molecule, such as dimethylol propionic acid and dimethylolbutanoic acid; polyester diols obtained by polycondensation of at least one of the low-molecular-weight diols with dicarboxylic acids such as adipic acid, sebacic acid, itaconic acid, maleic anhydride, terephthalic acid, and isophthalic acid; polyether diols such as polyethylene glycol, polypropylene glycol, and polytetramethylene ether diol; and 6-hydroxyhexanoic acid, polycaprolactone diol, polycarbonate diol, polybutadiene diol, hydrogenated polybutadiene diol, and polyacrylate diol. Only one type of the polyhydric alcohols may be independently used, or two or more types thereof may be used together.

The polyhydric alcohols preferably include a polyether diol from the viewpoint of further increasing compatibility and flexibility.

A content of the structural units derived from the polyhydric alcohol is preferably more than 75 mol% and less than 97 mol%, and more preferably 82 mol% to 95 mol% based on all structural units of the polyurethane.

The polyurethane preferably has a structural unit ($-CH_2CH_2O-$ or $-OCH_2CH_2-$) derived from ethylene oxide as a soft segment. Accordingly, the flexibility (elongation percentage) of the polyurethane can be controlled to be higher. In addition, the compatibility with the hydrophilic polymer can be further enhanced. Here, a content of the structural units derived from ethylene oxide is preferably more than 15 mol% and less than 45 mol%, and more preferably more than 20 mol% and less than 40 mol% based on all structural units of the polyurethane. The polyurethane having the structural units derived from ethylene oxide as the soft segment in such an amount can exhibit higher flexibility (elongation percentage). In addition, good compatibility with the hydrophilic polymer is exhibited. Therefore, by using such a polyurethane, the lubrication retaining property (sliding durability) and flexibility can be further improved.

As a method for producing the polyurethane, a known method, such as a method in which the polyhydric alcohol compound and the isocyanate compound react in an ether such as dioxane using a catalyst such as dibutyltin dilaurate, can be used in the same manner or with appropriate modifications.

The weight average molecular weight (Mw) of the polyurethane is preferably 100,000 or more. In addition, the weight average molecular weight of the polyurethane is preferably 5,000,000 or less. Here, "the weight average molecular weight" is measured by dissolving polyurethane (sample) in an appropriate solvent, using polystyrene as a standard substance, and performing gel permeation chromatography (GPC). The molecular weight of the polyurethane can also be calculated based on the type of the repeating unit and the number of the repeating units.

The polyurethane may be used in the form of being dispersed in water (in the form of an aqueous polyurethane dispersion). In this case, a content (solid content concentration) of the polyurethane in the aqueous polyurethane dispersion is, for example, 80 mass% or less, preferably 70 mass% or less, and more preferably 60 mass% or less. In addition, the content (solid content concentration) of the polyurethane in the aqueous polyurethane dispersion is, for example, 10 mass% or more, preferably 20 mass% or more, and more preferably 25 mass% or more. In an embodiment of the present invention, the content of the polyurethane in the aqueous dispersion is 10 mass% to 80 mass%, preferably 20 mass% to 70 mass%, and more preferably 25 mass% to 60 mass%.

When the polyurethane is used in the form of being dispersed in water, the average particle diameter (diameter) of the polyurethane in the aqueous polyurethane dispersion is preferably more than 50 nm, more preferably 60 nm or more, and particularly preferably more than 60 nm. In addition, the average particle diameter (diameter) of the polyurethane in the aqueous polyurethane dispersion is preferably 1000 nm or less, more preferably 800 nm or less, and particularly preferably 700 nm or less. That is, the average particle diameter of the polyurethane in the aqueous polyurethane dispersion is preferably more than 50 nm and 1000 nm or less, more preferably 60 nm to 800 nm, and particularly preferably more than 60 nm and 700 nm or less. In the present specification, the average particle diameter (diameter) of the polyurethane in the aqueous polyurethane dispersion is a value of a harmonic average particle diameter (nm) based on a scattered light intensity standard, which is calculated according to cumulant analysis by performing measurement at room temperature using a Nanotrac particle size distribution measurement device UPA-EX150 (manufactured by NIKKISO CO., LTD.) according to a light scattering method.

As the aqueous dispersion of the polyurethane resin as described above, a commercially available polyurethane aqueous polymer dispersion (polyurethane dispersion: PUD) may also be used as it is. Specifically, SUPERFLEX (registered trademark) 300 (non-yellowing isocyanate ester-ether-based polyurethane, elongation percentage: 1500%, Young's modulus: 5.4 $N/mm^2$, 100% modulus: 1.7 $N/mm^2$, EO content rate: more than 20 mol%, weight average molecular weight: 100,000 or more, average particle diameter in aqueous dispersion: 70 nm), SUPERFLEX (registered trademark) 500M (non-yellowing isocyanate ester-based polyurethane, elongation percentage: 1100%, Young's modulus: 319 $N/mm^2$, 100% modulus: 6.2 $N/mm^2$, EO content rate: more than 20 mol%, weight average molecular weight: 100,000 or more, average particle diameter in aqueous dispersion: 140 nm), SUPERFLEX (registered trademark) E-2000

(non-yellowing isocyanate ester-based polyurethane, elongation percentage: 1350%, Young's modulus: 11 N/mm$^2$, 100% modulus: 0.7 N/mm$^2$, EO content rate: more than 20 mol%, weight average molecular weight: 100,000 or more, average particle diameter in aqueous dispersion: 700 nm), and the like (all manufactured by DAIICHI KOGYO SEIYAKU CO., LTD.) can be used.

Only one type of the polyurethane or the aqueous polyurethane dispersion may be independently used, or two or more types thereof may be used together.

A mixing ratio of the polyurethane to the hydrophilic polymer in the surface lubricating layer is set, for example, such that the polyurethane is contained in a proportion of preferably 50 parts by mass to 1200 parts by mass, more preferably 100 parts by mass to 1000 parts by mass, and particularly preferably 200 parts by mass to 600 parts by mass based on 100 parts by mass of the hydrophilic polymer. With such a mixing ratio, the hydrophilic polymer and the polyurethane are more favorably compatible with each other, and elution of the hydrophilic polymer during contact with the aqueous liquid can be more effectively inhibited or prevented (lubrication retaining property (sliding durability) can be more improved). That is, in a preferable embodiment of the present invention, the polyurethane is contained in the surface lubricating layer in a proportion of 50 parts by mass to 1200 parts by mass based on 100 parts by mass of the polymer (hydrophilic polymer). In a more preferable embodiment of the present invention, the polyurethane is contained in the surface lubricating layer in a proportion of 100 parts by mass to 1000 parts by mass based on 100 parts by mass of the polymer (hydrophilic polymer). In a particularly preferable embodiment of the present invention, the polyurethane is contained in the surface lubricating layer in a proportion of 200 parts by mass to 600 parts by mass based on 100 parts by mass of the polymer (hydrophilic polymer). Here, the presence and mixing ratio of the hydrophilic polymer and the polyurethane in the surface lubricating layer can be checked and measured using a known analysis method such as FT-IR, XPS, and TOF-SIMS. For example, the presence and the mixing ratio of the hydrophilic polymer and the polyurethane in the surface lubricating layer can be checked by performing FT-IR measurement before and after the irradiation with active energy rays and comparing a ratio of a peak generated by bonds formed by the irradiation with the active energy ray to a peak generated by bonds which are unchanged.

In the present specification, the presence and the mixing ratio of the hydrophilic polymer and the polyurethane in the surface lubricating layer are evaluated by FT-IR using an FT-IR spectrophotometer (Nicolet iS50, manufactured by Thermo Fisher Scientific). Specifically, the surface lubricating layer or the medical device including the surface lubricating layer is set on a sample stage, and surface analysis is performed by ATR-IR. In the obtained IR chart, a peak in a vicinity of 1570 cm$^{-1}$ indicates C=O stretching vibration caused by carboxylates or the like. In addition, a peak in a vicinity of 1530 cm$^{-1}$ indicates CNH bending vibration caused by a polyurethane. Therefore, for example, in the IR chart in FIG. 2, when the peak in the vicinity of 1570 cm$^{-1}$ (a "peak A" in FIG. 2) is observed, it is indicated that the surface lubricating layer contains the hydrophilic polymer. In addition, when the peak in the vicinity of 1530 cm$^{-1}$ (a "peak B" in FIG. 2) is observed, it is indicated that the surface lubricating layer contains polyurethane.

In addition, in FIG. 2, an intersection (an "intersection A" in FIG. 2) of a tangent line A connecting a valley A and a valley A' present on the left and right of the peak A and a line vertically going down from the peak A is determined. Next, a distance ($X_A$ (mm)) between the peak A and the intersection A is measured. Similarly, an intersection (an "intersection B" in FIG. 2) of a tangent line B connecting a valley B and a valley B' present on the left and right of the peak B and a line vertically going down from the peak B is determined. Next, a distance ($X_B$ (mm)) between the peak B and the intersection B is measured. Based on the distance ($X_A$ (mm)) and the distance ($X_B$ (mm)), the mixing ratio of the polyurethane to the hydrophilic polymer in the surface lubricating layer (mixing ratio of the polyurethane to 100 parts by mass of the polymer (hydrophilic polymer)) is calculated by the following formula (A).

[Math 1]

Formula (A):

$$\text{Mixing ratio} = X_B\ (\text{mm}) \times 100 / X_A\ (\text{mm})$$

As described above, by using a combination of a polyurethane having a specific elongation percentage and Young's modulus and a hydrophilic polymer, the surface lubricating layer can exhibit higher lubrication retaining property (sliding durability) and flexibility under severe situations. In addition, such a surface lubricating layer can exhibit a higher lubricating property during contact with the aqueous liquid under severe situations.

(Other Components)

The surface lubricating layer may be constituted by only the hydrophilic polymer and the polyurethane, or may further contain other components in addition to the hydrophilic polymer and the polyurethane.

In an embodiment of the present invention, the surface lubricating layer is substantially constituted by a

hydrophilic polymer and a polyurethane. Here, "the surface lubricating layer is substantially constituted by a hydrophilic polymer and a polyurethane" means that a total content (in terms of solid content) of the hydrophilic polymer and the polyurethane in the surface lubricating layer is more than 95 mass% (upper limit: 100 mass%).

In an embodiment of the present invention, the surface lubricating layer is constituted by a hydrophilic polymer and a polyurethane (the total content (in terms of solid content) of the hydrophilic polymer and the polyurethane in the surface lubricating layer = 100 mass%).

In an embodiment of the present invention, the surface lubricating layer contains a hydrophilic polymer, a polyurethane, and other components. In the present embodiment, the other component is preferably a polyisocyanate. The isocyanate groups of the polyisocyanate are crosslinked to form a self-crosslinked product. The self-crosslinked product is also physically entangled (compatible) with the hydrophilic polymer in the surface lubricating layer. Therefore, elution of the hydrophilic polymer during expansion of the hydrophilic polymer due to wetting can be further inhibited or prevented. In addition, when the material constituting the base layer has an active hydrogen, the surface lubricating layer can be linked to the base layer via the polyisocyanate. Therefore, the lubrication retaining property (durability) under severe situations can be further improved by allowing the polyisocyanate to coexist. That is, in a preferable embodiment of the present invention, the surface lubricating layer further contains a polyisocyanate or a crosslinked product thereof. In the present specification, the "crosslinked product of a polyisocyanate" is intended to have a structure obtained by self-crosslinking the isocyanate groups (-N=C=O) of the polyisocyanate, or a structure that binds to an active hydrogen present in the base layer.

The polyisocyanate may be synthesized by a known method, or a commercially available product may be used. Examples of the commercially available product include: Elastron (registered trademark) series (for example, BN-69, BN-77, BN-27, and BN-04) (manufactured by DAIICHI KOGYO SEIYAKU CO., LTD.); Luxate HC series (for example, 1170 and 2170) (manufactured by ORIN CHEMICALS); Coronate series (for example, BI-301, 2507, 2554, 2512, 2513, 2515, and 2520) (manufactured by TOSOH CORPORATION); Burnock series (for example, D-500, D-550, and DB-980K) (manufactured by DIC Corporation); Takenate series (for example, WB-700, WB-720, WB-730, WB-920, XWB-72-K55, B-830, B-815, B-846, B-870, B-874, and B-882) (manufactured by MITSUI CHEMICALS, INC.); Sumidur series (for example, BL-3175, BL-4165, BL-1100, and BL-1265), Desmodur series (for example, TPLS-2957, TPLS-2062, TPLS-2078, and TPLS-2117), Desmotherm series (for example, 2170 and 2265), and Bayhydur series (for example, BL5140 and BL5235) (all manufactured by SUMIKA COVESTRO URETHANE CO.,LTD.); Blocked isocyanate series (for example, 7950, 7951, 7960, 7961, 7962, 7990, 7991, 7992, AquaBI200, AquaBI220) (manufactured by Baxenden); and Duranate series (for example, 17B-60P, TPA-B80E, MF-B60B, MF-K60B, SBB-70P, SBN-70D, SBF-70E, E402-B80B, and WM44-L70G (manufactured by ASAHI KASEI CORPORATION).

One type of the polyisocyanate may be independently used, or two or more types thereof may be used together.

When the surface lubricating layer further contains other components in addition to the hydrophilic polymer and the polyurethane, the mixing ratio of other components is, for example, preferably 5 parts by mass to 80 parts by mass, and more preferably 20 parts by mass to 60 parts by mass based on 100 parts by mass of the hydrophilic polymer. With such a mixing ratio, the lubrication retaining property can be further improved. In addition, the film strength of the surface lubricating layer can be further increased.

In addition, when the surface lubricating layer further contains other components in addition to the hydrophilic polymer and the polyurethane, the surface lubricating layer is preferably substantially constituted by the hydrophilic polymer, the polyurethane, and other components (particularly, a polyisocyanate). That is, in an embodiment of the present invention, the surface lubricating layer is substantially constituted by a hydrophilic polymer, a polyurethane, and other components. In a preferable embodiment of the present invention, the surface lubricating layer is substantially constituted by a hydrophilic polymer, a polyurethane, and a polyisocyanate. Here, "the surface lubricating layer is substantially constituted by a hydrophilic polymer, a polyurethane, and other components (or a polyisocyanate)" means that a total content (in terms of solid content) of the hydrophilic polymer, the polyurethane, and other components (or the polyisocyanate) in the surface lubricating layer is more than 95 mass% (upper limit: 100 mass%).

In an embodiment of the present invention, the surface lubricating layer is constituted by a hydrophilic polymer, a polyurethane, and other components (the total content (in terms of solid content) of the hydrophilic polymer, the polyurethane, and other components in the surface lubricating layer = 100 mass%). In a preferable embodiment of the present invention, the surface lubricating layer is constituted by a hydrophilic polymer, a polyurethane, and a polyisocyanate (the total content (in terms of solid content) of the hydrophilic polymer, the polyurethane, and the polyisocyanate in the surface lubricating layer = 100 mass%).

Alternatively, when the medical device such as a catheter is intended to be inserted into a lumen in a living body or a cavity, for example, a drug (a physiologically active substance) may be used as other components. Examples of the drug include an anticancer agent, an immunosuppressive drug, an antibiotic, an antirheumatic drug, an antithrombotic drug, an HMG-CoA reductase inhibitor, an ACE inhibitor, a calcium antagonist, an antihyperlipidemic agent drug, an integrin inhibitor, an antiallergic agent, an antioxidant, a GPIIb/IIIa antagonist, a retinoid, a flavonoid, a carotenoid, a lipid improving agent, a DNA synthesis inhibitor, a tyrosine kinase inhibitor, an antiplatelet drug, a vascular muscle growth inhibitor, an

anti-inflammatory drug, a tissue-derived biomaterial, an interferon, and a NO bio-generation promoting substance. In this case, the addition amount of the other components is not particularly limited, and an amount which is generally used is similarly applied. The addition amount of the other components is appropriately selected in consideration of severity of a disease for which the other components are to be applied, a body weight of a patient, and the like, and is finally determined by an attending physician.

The medical device as described above can exhibit an excellent lubrication retaining property (sliding durability), flexibility (particularly, flexibility of the distal end), and lubricating property even under severe situations. Therefore, the present medical device having the configuration can be favorably applied to a complicated lesion site.

[Method for Producing Medical Device]

In a second aspect, the invention relates to a method for producing a medical device according to claim 7.

According to the claimed method, the hydrophilic polymer, the polyurethane, and, if necessary, other components are added to and mixed with a solvent to prepare a coating liquid, and a base layer of a medical device is coated with the coating liquid to form a surface lubricating layer.

In addition, as described above, the present invention is characterized by using a polyurethane having specific characteristics. Such a polyurethane can be obtained by synthesizing (preparing) a polyurethane so as to have desired characteristics by using a known technique, or by measuring characteristics (elongation percentage, Young's modulus, etc.) of commercially available polyurethanes and selecting a polyurethane having desired characteristics. That is, the present invention provides a method for producing a medical device, and the method includes: preparing or selecting a polyurethane having an elongation percentage of 700% or more and a Young's modulus of 5.0 $N/mm^2$ or more; and preparing a coating liquid containing the polyurethane and a polymer having a carboxy group, or a salt or an ester thereof, and coating a base layer with the coating liquid to form a surface lubricating layer on the base layer.

As described above, the surface lubricating layer may be substantially constituted by the hydrophilic polymer and the polyurethane. That is, the present invention also provides a method for producing a medical device, and the method includes mixing only a polymer having a carboxy group or a salt or an ester thereof, a polyurethane having an elongation percentage of 700% or more and a Young's modulus of 5.0 $N/mm^2$ or more, and a solvent to prepare a coating liquid, and coating a base layer with the coating liquid to form a surface lubricating layer on the base layer. In addition, the present invention also provides a method for producing a medical device, and the method includes: preparing or selecting a polyurethane having an elongation percentage of 700% or more and a Young's modulus of 5.0 $N/mm^2$ or more; and mixing only the polyurethane, a polymer having a carboxy group or a salt or an ester thereof, and a solvent to prepare a coating liquid, and coating a base layer with the coating liquid to form a surface lubricating layer on the base layer.

Alternatively, as described above, the surface lubricating layer may be substantially constituted by the hydrophilic polymer, the polyurethane, and the polyisocyanate. That is, the present invention also provides a method for producing a medical device, and the method includes mixing only a polymer having a carboxy group or a salt or an ester thereof, a polyurethane having an elongation percentage of 700% or more and a Young's modulus of 5.0 $N/mm^2$ or more, a polyisocyanate, and a solvent to prepare a coating liquid, and coating a base layer with the coating liquid to form a surface lubricating layer on the base layer. In addition, the present invention also provides a method for producing a medical device, and the method includes: preparing or selecting a polyurethane having an elongation percentage of 700% or more and a Young's modulus of 5.0 $N/mm^2$ or more; and mixing only the polyurethane, a polymer having a carboxy group or a salt or an ester thereof, a polyisocyanate, and a solvent to prepare a coating liquid, and coating a base layer with the coating liquid to form a surface lubricating layer on the base layer.

(Step of Preparing or Selecting Polyurethane)

Here, a polyurethane is synthesized (prepared) so as to have an elongation percentage of 700% or more and a Young's modulus of 5.0 $N/mm^2$ or more. The polyurethane having the above specific characteristics can be produced by using a known technique or appropriately modifying a known technique.

Alternatively, commercially available polyurethanes may be purchased and respectively measured for the characteristics (elongation percentage, Young's modulus, etc.), and a polyurethane having an elongation percentage of 700% or more and a Young's modulus of 5.0 $N/mm^2$ or more may be selected.

(Step of Coating Surface Lubricating Layer)

Here, a hydrophilic polymer, the polyurethane (the polyurethane prepared or selected as described above), and other components as necessary (for example, a polyisocyanate) are dissolved or dispersed in a solvent to prepare a coating liquid, and a base layer is coated with the coating liquid. Here, the solvent that can be used for preparing the coating liquid is not particularly limited as long as it can dissolve/disperse desired components. Specific examples thereof include,

but are not limited to, water; alcohols such as methanol, ethanol, isopropanol, and ethylene glycol; ketones such as acetone, methyl ethyl ketone, and cyclohexanone; esters such as ethyl acetate; halides such as chloroform; alkanes such as hexane; ethers such as tetrahydrofuran (THF) and butyl ether; aromatics such as benzene and toluene; amides such as N,N-dimethylformamide (DMF); and sulfoxides such as dimethyl sulfoxide. One type of them may be independently used, or two or more types thereof may be used together. Here, the hydrophilic polymer and the polyurethane may be added to a solvent together, or may be sequentially added to the same solvent (the polyurethane may be added after the hydrophilic polymer is added or the hydrophilic polymer may be added after the polyurethane is added). Alternatively, the hydrophilic polymer and the polyurethane may be dissolved or dispersed in respective solvents, followed by mixing. When the hydrophilic polymer and the polyurethane are dissolved or dispersed in respective solvents, the solvents may be the same as or different from each other, and it is preferable that the hydrophilic polymer and the polyurethane can be dissolved in the same solvents or in a uniform manner in consideration of ease of operation.

In addition, when the coating liquid is prepared using the hydrophilic polymer, the polyurethane, and other components (for example, a polyisocyanate), the hydrophilic polymer, the polyurethane, and other components may also be added to a solvent together, or may also be added to the same solvent in any order (for example, other components may be added after the hydrophilic polymer and the polyurethane are added; and the hydrophilic polymer, the polyurethane, and other components are added in this order). Alternatively, the hydrophilic polymer, the polyurethane, and other components may also be dissolved or dispersed in respective solvents, followed by mixing. When the hydrophilic polymer, the polyurethane, and other components are dissolved or dispersed in respective solvents, the solvents may be the same as or different from each other, and it is preferable that the hydrophilic polymer, the polyurethane and other components can be dissolved in the same solvents or in a uniform manner in consideration of ease of operation.

After the hydrophilic polymer, the polyurethane, and if necessary, other components (for example, a polyisocyanate) are added to the solvent, stirring may be performed if necessary in order to promote the dissolution or dispersion.

A concentration of the hydrophilic polymer, the polyurethane, and if necessary, other components (for example, a polyisocyanate) in the coating liquid is such a concentration that a total solid content concentration of the coating liquid is preferably 0.1 mass% to 50 mass%, and more preferably 0.5 mass% to 10 mass%. Within such a range, the coating liquid is excellent in terms of coatability and production efficiency. A mixing proportion of the hydrophilic polymer, the polyurethane, and if necessary, other components in the coating liquid is preferably the same as that described in the section of the above surface lubricating layer.

A coating amount of the coating liquid is not particularly limited, and is preferably such an amount that the above thickness of the surface lubricating layer is obtained.

Before the coating liquid is applied, the surface of the base layer may be treated in advance by an ultraviolet irradiation treatment, a plasma treatment, a corona discharge treatment, a flame treatment, an oxidation treatment, a silane coupling treatment, a phosphate coupling treatment, a surfactant treatment, or the like. When the solvent in the coating liquid is only water, it is difficult to coat the surface of the hydrophobic base layer with the coating liquid. The surface of the base layer becomes hydrophilic by subjecting the surface of the base layer to a plasma treatment or a surfactant treatment. Accordingly, the wettability of the coating liquid to the surface of the base layer is improved, and a uniform surface lubricating layer can be formed.

The method for coating the surface of the base layer with the coating liquid is not particularly limited, and known methods according to the related art, such as a coating/printing method, an immersion method (a dipping method and a dip coating method), an atomization method (a spraying method), a spin coating method, a mixed solution-impregnated sponge coating method, a bar coating method (for example, a coating method using a wire bar), a die coating method, a reverse coating method, a comma coating method, a gravure coating method, and a doctor-knife method, can be applied.

When the surface lubricating layer is formed on a thin and narrow inner surface of a catheter, an injection needle, or the like, the base layer may be immersed in a coating liquid and defoaming is performed while reducing a pressure in the system. By performing defoaming while reducing the pressure, the solution can be quickly permeated into the thin and narrow inner surface and formation of the surface lubricating layer can be promoted.

When the surface lubricating layer is formed only on a part of the base layer, the surface lubricating layer can be formed on a desired surface site in the base layer by immersing only a part of the base layer in the coating liquid and coating the part of the base layer with the coating liquid.

When it is difficult to dip only a part of the base layer in the coating liquid, a surface portion of the base layer, which does not need to be provided with the surface lubricating layer, is protected (covered) with an appropriate detachable (attachable and detachable) member or material in advance. The base layer is immersed in the coating liquid and is coated with the coating liquid, followed by removing the protective member (material) on the surface portion of the base layer, which does not need to be provided with the surface lubricating layer, and then a reaction is caused with a heat treatment or the like, so that the surface lubricating layer can be formed on a desired surface site on the base layer. However, in the present invention, the method for forming the surface lubricating layer is not limited, and the surface lubricating layer can be formed by appropriately using a known method according to the related art. For example, when it is difficult to dip only a part of the base layer in the coating liquid, another coating method (for example, a method in which a predetermined surface

portion of a medical device is coated with the coating liquid using a coating device such as a spray device, a bar coater, a die coater, a reverse coater, a comma coater, a gravure coater, a spray coater, and a doctor knife) may be applied instead of the immersion method. When both an outer surface and an inner surface of a cylindrical device need to have a surface lubricating layer due to a structure of a medical device, an immersion method (dipping method) is preferably used in view that both the outer surface and the inner surface can be coated at once.

(Step of Drying Surface Lubricating Layer)

As described above, it is preferable to dry (perform a heat treatment on) a coating film after coating the base layer with the coating liquid. The drying conditions are not particularly limited as long as the solvent can be removed from the coating film, and the coating film may be dried by an appropriate drying unit (device), may be subjected to a warm air treatment using a dryer or the like, or may be naturally dried. In addition, the pressure condition during drying is not limited at all, and the drying may be performed under a normal pressure (atmospheric pressure) or under an increased pressure or a reduced pressure. As the drying unit (device), for example, an oven (drying furnace) and a vacuum dryer can be used, and in the case of the natural drying, no particular drying unit (device) is required.

The drying temperature and the drying time are not particularly limited, and are appropriately selected according to the type of the solvent and the heat-resistant temperature of the base material. The drying temperature is preferably 40°C to 200°C, and more preferably 70°C to 150°C. In addition, the drying time is preferably 5 minutes to 24 hours, and more preferably 20 minutes to 5 hours.

The drying step may be performed only once, or may be performed a plurality of times under different drying conditions.

As described above, the surface lubricating layer is formed on the base layer. The film provided on the base layer may be washed after the above drying step.

The medical device obtained by such a method can exhibit an excellent lubrication retaining property (sliding durability), excellent flexibility (particularly, flexibility of the distal end), and an excellent lubricating property even under severe situations. Therefore, the present medical device can also be applied to a complicated lesion site.

[Use of Medical Device]

The medical device according to the present invention is used in contact with a body fluid, blood, or the like, and has a surface having a lubricating property in an aqueous liquid such as a body fluid or a physiological saline. The operability of the medical device can be improved, and the damage to a tissue mucosa generated by the medical device can be reduced. Specific examples thereof include a catheter, a stent, and a guide wire to be used in a blood vessel. That is, the medical device according to an embodiment of the present invention is a catheter or a guide wire. In addition, the following medical devices are shown.

(a) Catheters to be inserted or indwelled in a digestive organ orally or nasally, such as a gastric tube catheter, a feeding catheter, and a tube-feeding tube;

(b) Catheters to be inserted or indwelled into an airway or a trachea orally or nasally, such as an oxygen catheter, an oxygen cannula, a tube or cuff of an endotracheal tube, a tube or cuff of a tracheostomy tube, and an endotracheal suctioning catheter;

(c) Catheters to be inserted or indwelled in a urethra or a ureter, such as a urethral catheter, a urinary catheter, and a catheter or a balloon in a urethral balloon catheter;

(d) Catheters to be inserted or indwelled in various lumina in a living body, organs, or tissues, such as a suction catheter, a discharge catheter, and a rectal catheter;

(e) Catheters to be inserted or indwelled in blood vessels such as an indwelling needle, an IVH catheter, a thermodilution catheter, an angiographic catheter, a vasodilation catheter, a dilator, and an introducer, or guidewires or stylets for these catheters;

(f) An artificial trachea, an artificial bronchi, or the like; and

(g) A medical device for extracorporeal circulation treatment (an artificial lung, an artificial heart, an artificial kidney, etc.) and circuits thereof.

Although the embodiment of the present invention has been described in detail, it is to be understood that the present invention is not limited thereto and should be interpreted by the appended claims.

[Examples]

Effects of the present invention will be described using the following Examples and Comparative Examples. However, the technical scope of the present invention is not limited to only the following Examples. In the following Examples, unless otherwise specified, the operation was performed at room temperature (25°C). In addition, unless otherwise specified, "%" and "part(s)" mean "mass%" and "part(s) by mass", respectively.

Example 1

To 60 g of water was added 0.1 g of sodium polyacrylate (manufactured by FUJI FILM WAKO PURE CHEMICAL INDUSTRIES, LTD., polymerization degree: 30,000 to 40,000) followed by stirring for 2 hours, and an aqueous sodium polyacrylate solution was prepared. With the aqueous sodium polyacrylate solution was mixed an aqueous dispersion of a non-yellowing isocyanate ester-based polyurethane (polyurethane concentration: 50 mass%) (SUPERFLEX (registered trademark) E-2000, manufactured by DAIICHI KOGYO SEIYAKU CO., LTD., elongation percentage: 1350%, Young's modulus: 11 $N/mm^2$, 100% modulus: 0.7 $N/mm^2$, EO content rate: more than 20 mol%, weight average molecular weight: 100,000 or more, average particle diameter in the aqueous dispersion: 700 nm; hereinafter, also referred to as "polyurethane E2000" or "E2000") such that a mass ratio (solid content ratio) of sodium polyacrylate/E2000 was 100/400, followed by stirring for 10 minutes, and a coating liquid in which a total solid content concentration of resin components (sodium polyacrylate and E2000) was 0.8 mass% was obtained.

Next, a polyethylene terephthalate (PET) film (Cosmo Shine (registered trademark) A4100, manufactured by TOYOBO CO., LTD., thickness: 100 μm) was coated with the above obtained coating liquid by a wire bar under an environment of 25°C and a relative humidity of 45% RH such that a film thickness (dry film thickness) was 2 μm to 3 μm, thereby forming a coating film on the PET film. Thereafter, the film on which the coating film was formed was dried at 80°C for 30 minutes and then at 100°C for 30 minutes in a drying furnace to form a lubricating layer (dry film thickness: 2 μm to 3 μm) containing sodium polyacrylate and E2000 (sample 1) on the PET film.

Example 2

A lubricating layer (dry film thickness: 2 μm to 3 μm) containing sodium polyacrylate and SF300 (sample 2) was formed on a PET film in the same manner as in Example 1, except that a non-yellowing isocyanate ester-ether-based polyurethane (SUPERFLEX (registered trademark) 300, manufactured by DAIICHI KOGYO SEIYAKU CO., LTD., elongation percentage: 1500%, Young's modulus: 5.4 $N/mm^2$, 100% modulus: 1.7 $N/mm^2$, EO content rate: more than 20 mol%, weight average molecular weight: 100,000 or more, average particle diameter in the aqueous dispersion: 70 nm; hereinafter, also referred to as "polyurethane SF300" or "SF300") was used instead of the polyurethane E2000 in Example 1.

Example 3

To 40 g of water was added 0.05 g of sodium hyaluronate (manufactured by TOKYO CHEMICAL INDUSTRY CO., LTD., product code: H0603, derived from a cock's comb, molecular weight: 350,000 or more (in terms of dry product)), followed by stirring for 2 hours, and an aqueous sodium hyaluronate solution was prepared. The polyurethane SF300 was mixed with the aqueous sodium hyaluronate solution such that a mass ratio (solid content ratio) of sodium hyaluronate/SF300 was 100/400, followed by stirring for 10 minutes, and a coating liquid in which a total solid content concentration of resin components (sodium hyaluronate and SF300) was 0.6 mass% was obtained.

Next, a PET film (Cosmo Shine (registered trademark) A4100, manufactured by TOYOBO CO., LTD., thickness: 100 μm) was coated with the above obtained coating liquid by a wire bar under an environment of 25°C and a relative humidity of 45% RH such that a film thickness (dry film thickness) was 2 μm to 3 μm, thereby forming a coating film on the PET film. Thereafter, the film on which the coating film was formed was dried at 80°C for 30 minutes and then at 100°C for 30 minutes in a drying furnace to form a lubricating layer (dry film thickness: 2 μm to 3 μm) containing sodium hyaluronate and SF300 (sample 3) on the PET film.

Example 4

A half ethyl ester (degree of esterification: 40% to 50%) of a methyl vinyl ether maleic anhydride copolymer (GANTREZ AN-169, manufactured by ISP) was added to acetone so as to have a concentration of 2.6 mass%, and dissolved by stirring for 1 hour. Next, the obtained solution was mixed with an aqueous sodium hydrogen carbonate solution in an amount such that sodium hydrogen carbonate was 1.05 mol equivalent per unit of the half ethyl ester of the methyl vinyl ether maleic anhydride copolymer, followed by stirring for 1 hour. Accordingly, the carboxylic acid groups in the

half ethyl ester of the methyl vinyl ether maleic anhydride copolymer are all neutralized with sodium carboxylate (the hydrophilic polymer is constituted by the following structural units). The hydrophilic polymer obtained here is hereinafter referred to as a "hydrophilic polymer 1".

[Chem. 8]

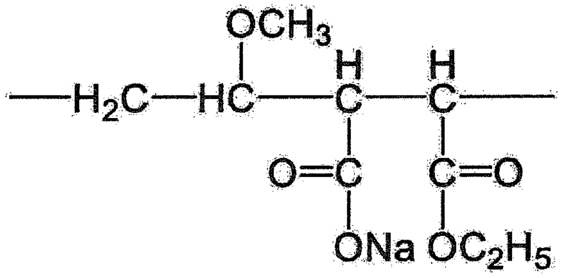

The hydrophilic polymer 1 obtained as described above and the polyurethane E2000 were mixed such that a mass ratio (solid content ratio) of the hydrophilic polymer 1/E2000 was 100/400. The mixture was adjusted such that a total solid content concentration of resin components (the hydrophilic polymer 1 and E2000) was 6.0 mass% and a mixing ratio (mass ratio) of acetone to water was 50/50, and the mixture was stirred for 1 hour to obtain a coating liquid.

Next, a PET film (Cosmo Shine (registered trademark) A4100, manufactured by TOYOBO CO., LTD., thickness: 100 μm) was coated with the above obtained coating liquid by a wire bar under an environment of 25°C and a relative humidity of 45% RH such that a film thickness (dry film thickness) was 2 μm to 3 μm, thereby forming a coating film on the PET film. Thereafter, the film on which the coating film was formed was dried at 80°C for 30 minutes and then at 100°C for 30 minutes in a drying furnace to form a lubricating layer (dry film thickness: 2 μm to 3 μm) containing the hydrophilic polymer 1 and E2000 (sample 4) on the PET film.

Comparative Example 1

Sodium polyacrylate (manufactured by FUJI FILM WAKO PURE CHEMICAL INDUSTRIES, LTD., polymerization degree: 30,000 to 40,000) was added to water so as to have a solid content concentration of 0.2 mass%, and was dissolved by stirring for 2 hours. Next, in order to prevent cissing during coating, a nonionic surfactant (Noigen (registered trademark) EA87, polyoxyethylene styryl phenyl ether, manufactured by DAIICHI KOGYO SEIYAKU CO., LTD.) was added in a proportion of 5 mass% based on sodium polyacrylate, and a coating liquid was obtained.

Next, a PET film (Cosmo Shine (registered trademark) A4100, manufactured by TOYOBO CO., LTD., thickness: 100 μm) was coated with the above obtained coating liquid by a wire bar under an environment of 25°C and a relative humidity of 45% RH such that a film thickness (dry film thickness) was 2 μm to 3 μm, thereby forming a coating film on the PET film. Thereafter, the film on which the coating film was formed was dried at 80°C for 30 minutes and then at 100°C for 30 minutes in a drying furnace to form a lubricating layer (dry film thickness: 2 μm to 3 μm) containing sodium polyacrylate (comparative sample 1) on the PET film.

Comparative Example 2

Polyacrylamide (manufactured by TOKYO CHEMICAL INDUSTRY CO., LTD., Mw = 400,000 to 800,000) and the polyurethane E2000 were mixed such that a mass ratio (solid content ratio) of polyacrylamide/E2000 was 100/400. The mixture was added to water such that a total solid content concentration of resin components (polyacrylamide and E2000) was 8.6 mass%, and the mixture was stirred for 2 hours to obtain a coating liquid.

Next, a PET film (Cosmo Shine (registered trademark) A4100, manufactured by TOYOBO CO., LTD., thickness: 100 μm) was coated with the above obtained coating liquid by a wire bar under an environment of 25°C and a relative humidity of 45% RH such that a film thickness (dry film thickness) was 2 μm to 3 μm, thereby forming a coating film on the PET film. Thereafter, the film on which the coating film was formed was dried at 80°C for 30 minutes and then at 100°C for 30 minutes in a drying furnace to form a lubricating layer (dry film thickness: 2 μm to 3 μm) containing polyacrylamide and E2000 (comparative sample 2) on the PET film.

Comparative Example 3

A lubricating layer (dry film thickness: 2 μm to 3 μm) containing polyacrylamide and SF300 (comparative sample 3) was formed on a PET film in the same manner as in Comparative Example 2, except that the polyurethane SF300 was

used instead of the polyurethane E2000 in Comparative Example 2.

Comparative Example 4

Polyvinylpyrrolidone (K90, manufactured by TOKYO CHEMICAL INDUSTRY CO., LTD., weight average molecular weight = 360,000) and the polyurethane E2000 were mixed such that a mass ratio (solid content ratio) of polyvinylpyrrolidone/E2000 was 100/400. The mixture was added to water such that a total solid content concentration of resin components (polyvinylpyrrolidone and E2000) was 10 mass%, and the mixture was stirred for 2 hours to obtain a coating liquid.

Next, a PET film (Cosmo Shine (registered trademark) A4100, manufactured by TOYOBO CO., LTD., thickness: 100 μm) was coated with the above obtained coating liquid by a wire bar under an environment of 25°C and a relative humidity of 45% RH such that a film thickness (dry film thickness) was 2 μm to 3 μm, thereby forming a coating film on the PET film. Thereafter, the film on which the coating film was formed was dried at 80°C for 30 minutes and then at 100°C for 30 minutes in a drying furnace to form a lubricating layer (dry film thickness: 2 μm to 3 μm) containing polyvinylpyrrolidone and E2000 (comparative sample 4) on the PET film.

Comparative Example 5

A lubricating layer (dry film thickness: 2 μm to 3 μm) containing polyvinylpyrrolidone and SF300 (comparative sample 5) was formed on a PET film in the same manner as in Comparative Example 4, except that the polyurethane SF300 was used instead of polyurethane E2000 in Comparative Example 4.

[Sliding Durability Evaluation 1]

Regarding the samples 1 to 4 in the above Examples 1 to 4 and the comparative samples 1 to 5 in Comparative Examples 1 to 5, the sliding durability of the lubricating layer was evaluated by using a friction tester (Handy Tribo Master TL201, manufactured by TRINITY LAB INC.) 10 shown in FIG. 1 in accordance with the following method.

That is, the above sample 3 was fixed in a petri dish 2 with the lubricating layer surface of the sample 3 facing upward, and was immersed in water 1 having a height at which the entire sample 3 was immersed. The petri dish 2 is mounted on a moving table 6 of the friction tester 10 shown in FIG.1. A terminal (φ10 mm) 4 made of a hydrogenated styrene-based thermoplastic elastomer (SEBS) was brought into contact with the sample 3, and a load 5 of 100 g was applied onto the terminal. Under the setting of a sliding distance of 15 mm and a sliding speed of 16.7 mm/sec, the moving table 6 was horizontally reciprocated 10 times, and the sliding resistance values (gf) on the first lap, the fifth lap, and the tenth lap were measured. The results are shown in the following Table 1. In the following Table 1, a case where the sliding resistance value on the tenth lap is less than 1.0 gf is indicated by "A", a case where the sliding resistance value on the tenth lap is 1.0 gf or more and less than 5.0 gf is indicated by "B", and a case where the sliding resistance value on the tenth lap is 5.0 gf or more is indicated by "C".

[Table 1]

| | Lubricating layer | | Sliding durability |
|---|---|---|---|
| | Hydrophilic polymer | Polyurethane | |
| Example 1 | Sodium polyacrylate | E2000 | B |
| Example 2 | Sodium polyacrylate | SF300 | A |
| Example 3 | Sodium hyaluronate | SF300 | B |
| Example 4 | Hydrophilic polymer 1 | E2000 | A |
| Comparative Example 1 | Sodium polyacrylate | - | C |
| Comparative Example 2 | Polyacrylamide | E2000 | C |
| Comparative Example 3 | Polyacrylamide | SF300 | C |
| Comparative Example 4 | Polyvinylpyrrolidone | E2000 | C |
| Comparative Example 5 | Polyvinylpyrrolidone | SF300 | C |

From the results in Table 1, it can be understood that the sliding durability can be significantly improved by using a polymer having a carboxy group or the like as the hydrophilic polymer.

Example 5

The hydrophilic polymer 1 was obtained in the same manner as in Example 4.

The hydrophilic polymer 1 and a non-yellowing isocyanate ester-based polyurethane (SUPERFLEX (registered trademark) 500M, manufactured by DAIICHI KOGYO SEIYAKU CO., LTD., elongation percentage: 1100%, Young's modulus: 319 N/mm$^2$, 100% modulus: 6.2 N/mm$^2$, EO content rate: more than 20 mol%, weight average molecular weight: 100,000 or more, average particle diameter in the aqueous dispersion: 140 nm; hereinafter, also referred to as "polyurethane SF500M" or "500M") were mixed such that a mass ratio (solid content ratio) of the hydrophilic polymer 1/500M was 100/400. The mixture was adjusted such that a total solid content concentration of resin components (the hydrophilic polymer 1 and 500M) was 6.0 mass% and a mixing ratio (mass ratio) of acetone to water was 50/50, and the mixture was stirred for 1 hour to obtain a coating liquid.

A Ni-Ti wire (outer diameter = 0.34 mm) was coated with a thermoplastic polyurethane resin in a thickness of about 35 μm to obtain a coated wire 1 (outer diameter = 0.41 mm) in which a polyurethane resin film (polyurethane underlayer) (thickness: about 35 μm) was formed on the wire. Next, the coated wire 1 was immersed in a tetrahydrofuran (THF) solution (PVC concentration = 10 mass%) of polyvinyl chloride (PVC) for 5 seconds under an environment of 25°C and a relative humidity of 45% RH, then pulled up at a velocity of 50 mm/sec, and naturally dried for 10 minutes, and a PVC film (PVC intermediate layer) (thickness: about 4 μm) was formed on a surface of the coated wire 1 (base material wire 1).

The base material wire 1 was immersed in the coating liquid prepared as described above for 5 seconds, then pulled up at a velocity of 50 mm/sec, and dried for 2 hours in a drying furnace maintained at 120°C, and a lubricating layer (dry film thickness: 4 μm) was formed on the PVC intermediate layer. Accordingly, a wire (sample 5) in which the lubricating layer containing the hydrophilic polymer 1 and 500M was formed on the PVC intermediate layer was obtained.

Example 6

A lubricating layer (dry film thickness: 4 μm) was formed on a PVC intermediate layer in the same manner as in Example 5, except that the polyurethane SF300 was used instead of the polyurethane 500M in Example 5. Accordingly, a wire (sample 6) in which the lubricating layer containing the hydrophilic polymer 1 and SF300 was formed on the PVC intermediate layer was obtained.

Example 7

A lubricating layer (dry film thickness: 4 μm) was formed on a PVC intermediate layer in the same manner as in Example 5, except that the polyurethane E2000 was used instead of the polyurethane 500M in Example 5. Accordingly, a wire (sample 7) in which the lubricating layer containing the hydrophilic polymer 1 and E2000 was formed on the PVC intermediate layer was obtained.

Comparative Example 6

A lubricating layer (dry film thickness: 4 μm) was formed on a PVC intermediate layer in the same manner as in Example 5, except that a non-yellowing isocyanate ester-ether-based polyurethane (SUPERFLEX (registered trademark) 150, manufactured by DAIICHI KOGYO SEIYAKU CO., LTD., elongation percentage: 330%, Young's modulus: 807 N/mm$^2$, 100% modulus: 19.0 N/mm$^2$, EO content rate: less than 15 mol%; hereinafter, also referred to as "polyurethane SF150" or "SF 150") was used instead of the polyurethane 500M in Example 5. Accordingly, a wire (comparative sample 6) in which the lubricating layer containing the hydrophilic polymer 1 and SF150 was formed on the PVC intermediate layer was obtained.

Comparative Example 7

A lubricating layer (dry film thickness: 4 μm) was formed on a PVC intermediate layer in the same manner as in Example 5, except that a non-yellowing isocyanate ester-based polyurethane (SUPERFLEX (registered trademark) 210, manufactured by DAIICHI KOGYO SEIYAKU CO., LTD., elongation percentage: 5%, Young's modulus: 1396 N/mm$^2$, 100% modulus: -; hereinafter, also referred to as "polyurethane SF210" or "SF210") was used instead of the polyurethane 500M in Example 5. Accordingly, a wire (comparative sample 7) in which the lubricating layer containing the hydrophilic polymer 1 and SF210 was formed on the PVC intermediate layer was obtained.

Comparative Example 8

A lubricating layer (dry film thickness: 4 μm) was formed on a PVC intermediate layer in the same manner as in

Example 5, except that a non-yellowing isocyanate ester-based polyurethane (SUPERFLEX (registered trademark) 740, manufactured by DAIICHI KOGYO SEIYAKU CO., LTD., elongation percentage: 1300%, Young's modulus: 0.3 $N/mm^2$, 100% modulus: 1.0 $N/mm^2$; hereinafter, also referred to as "polyurethane SF740" or "SF740") was used instead of the polyurethane 500M in Example 5. Accordingly, a wire (comparative sample 8) in which the lubricating layer containing the hydrophilic polymer 1 and SF740 was formed on the PVC intermediate layer was obtained.

Comparative Example 9

A lubricant layer (dry film thickness: 4 μm) was formed on the PVC intermediate layer in the same manner as in Example 5 except that a non-yellowing isocyanate carbonate-based polyurethane (SUPERFLEX (registered trademark) 470, manufactured by DAIICHI KOGYO SEIYAKU CO., LTD., elongation percentage: 640%, Young's modulus: 13 $N/mm^2$, 100% modulus: 2.5 $N/mm^2$, weight average molecular weight: 100,000 or more, average particle diameter in the aqueous dispersion: 50 nm; hereinafter, also referred to as "polyurethane SF470" or "SF470") was used instead of the polyurethane 500M in Example 5. Accordingly, a wire (comparative sample 9) in which the lubricating layer containing the hydrophilic polymer 1 and SF740 was formed on the PVC intermediate layer was obtained.

[Sliding Durability Evaluation 2]

Regarding the above samples 5 to 7 in Examples 5 to 7 and the comparative samples 6 to 9 in Comparative Examples 6 to 9, the sliding durability of the lubricating layer was evaluated by using the friction tester (Handy Tribo Master TL201, manufactured by TRINITY LAB INC.) 10 shown in FIG. 1 in accordance with the following method.

That is, the above sample 3 was fixed in the petri dish 2 with the lubricating layer surface of the sample 3 facing upward, and was immersed in the water 1 having a height at which the entire sample 3 was immersed. The petri dish 2 was mounted on the moving table 6 of the friction tester 10 shown in FIG.1. The terminal (φ10 mm) 4 made of a hydrogenated styrene-based thermoplastic elastomer (SEBS) was brought into contact with the sample 3, and the load 5 of 100 g was applied onto the terminal. Under the setting of a sliding distance of 15 mm and a sliding speed of 16.7 mm/sec, the moving table 6 was horizontally reciprocated 100 times, and the sliding resistance values (gf) on the first lap, the fifth lap, the tenth lap, the 30th lap, the 50th lap, and the 100th lap were measured. The results are shown in the following Table 2. In the following Table 2, a case where an increase in the resistance value from the first lap to the 100th lap (= (sliding resistance value on 100th lap) - (sliding resistance value on first lap)) is less than 1 gf, and the resistance value on the 100th lap is less than 2.5 gf is indicated as "A", a case where an increase in the resistance value from the first lap to the 50th lap (= (sliding resistance value on 50th lap) - (sliding resistance value on first lap)) is less than 1 gf, the resistance value on the 50th lap is less than 2.5 gf, and the resistance value on the 100th lap is 2.5 gf or more is indicated as "B", a case where an increase in the resistance value from the first lap to the 10th lap (= (sliding resistance value on tenth lap) - (sliding resistance value on first lap)) is less than 1 gf, the resistance value on the 10th lap is less than 2.5 gf, and the resistance value on the 50th lap is 2.5 gf or more is indicated as "C", and a case where the sliding resistance value on the 10th lap is 2.5 gf or more is indicated as "D".

[Table 2]

| | Lubricating layer | | | | | Sliding durability |
|---|---|---|---|---|---|---|
| | | Polyurethane | | | | |
| | Hydrophilic polymer | Type | Elongation percentage (%) | Young's modulus ($N/mm^2$) | 100% modulus ($N/mm^2$) | |
| Example 5 | Hydrophilic polymer 1 | 500M | 1100 | 319 | 6.2 | B |
| Example 6 | Hydrophilic polymer 1 | SF300 | 1500 | 5.4 | 1.7 | A |
| Example 7 | Hydrophilic polymer 1 | E2000 | 1350 | 11 | 0.7 | A |
| Comparative Example 6 | Hydrophilic polymer 1 | SF150 | 330 | 807 | 19.0 | D |
| Comparative Example 7 | Hydrophilic polymer 1 | SF210 | 5 | 1396 | - | D |
| Comparative Example 8 | Hydrophilic polymer 1 | SF740 | 1300 | 0.3 | 1.0 | D |
| Comparative Example 9 | Hydrophilic polymer 1 | SF470 | 640 | 13 | 2.5 | D |

From the results in Table 2, it can be understood that the surface lubricating layer can exhibit excellent sliding

durability by using a polyurethane having an elongation percentage of 700% or more and a Young's modulus of 5.0 $N/mm^2$ or more. On the other hand, it can be seen that Comparative Examples 6, 7, and 9 in which the elongation percentage is out of the range in the present invention and Comparative Example 8 in which the Young's modulus is out of the range in the present invention are inferior in the sliding durability.

In particular, in Samples 6 and 7 in Examples 6 and 7, the sliding resistance values from the first lap to the 100th lap were less than 2.0 gf, which indicates a very excellent lubrication retaining property (sliding durability). It is presumed that this is because the polyurethane used in the samples 6 and 7 can favorably follow the hydrophilic polymer due to the swelling of the hydrophilic polymer because the polyurethane has a high elongation percentage and a low 100% modulus, and the film strength of the surface lubricating layer can be more maintained with respect to the sliding due to the high Young's modulus. In the comparative sample 9 in Comparative Example 9, the sliding resistance value on the tenth lap was 2.5 gf or more, but an increase in the resistance value from the first lap to the tenth lap (= (sliding resistance value on tenth lap) - (sliding resistance value on first lap)) was less than 1 gf, and the resistance value on the tenth lap was less than 4 gf. As compared with other comparative samples 6 to 8 [increase in resistance value (= (sliding resistance value on tenth lap) - (sliding resistance value on first lap)) = more than 5 gf, and resistance value on tenth lap = more than 10 gf], the sliding durability of the surface lubricating layer in the comparative sample 9 was excellent, and the sliding resistance value thereof was low.

Example 8

A lubricating layer (dry film thickness: 4 μm) was formed on a PVC intermediate layer in the same manner as in Example 7, except that the hydrophilic polymer 1 and the polyurethane E2000 were mixed such that the mass ratio (solid content ratio) of the hydrophilic polymer 1/E2000 was changed to 100/50 as compared with that in Example 7. Accordingly, a wire (sample 8) in which the lubricating layer containing the hydrophilic polymer 1 and E2000 at a mixing ratio of 100/50 was formed on the PVC intermediate layer was obtained.

Example 9

A lubricating layer (dry film thickness: 4 μm) was formed on a PVC intermediate layer in the same manner as in Example 7, except that the hydrophilic polymer 1 and the polyurethane E2000 were mixed such that the mass ratio (solid content ratio) of the hydrophilic polymer 1/E2000 was changed to 100/100 as compared with that in Example 7. Accordingly, a wire (sample 9) in which the lubricating layer containing the hydrophilic polymer 1 and E2000 at a mixing ratio of 100/100 was formed on the PVC intermediate layer was obtained.

Example 10

A lubricating layer (dry film thickness: 4 μm) was formed on a PVC intermediate layer in the same manner as in Example 7, except that the hydrophilic polymer 1 and the polyurethane E2000 were mixed such that the mass ratio (solid content ratio) of the hydrophilic polymer 1/E2000 was changed to 100/200 as compared with that in Example 7. Accordingly, a wire (sample 10) in which the lubricating layer containing the hydrophilic polymer 1 and E2000 at a mixing ratio of 100/200 was formed on the PVC intermediate layer was obtained.

Example 11

A lubricating layer (dry film thickness: 4 μm) was formed on a PVC intermediate layer in the same manner as in Example 7, except that the hydrophilic polymer 1 and the polyurethane E2000 were mixed such that the mass ratio (solid content ratio) of the hydrophilic polymer 1/E2000 was changed to 100/600 as compared with that in Example 7. Accordingly, a wire (sample 11) in which the lubricating layer containing the hydrophilic polymer 1 and E2000 at a mixing ratio of 100/600 was formed on the PVC intermediate layer was obtained.

Example 12

A lubricating layer (dry film thickness: 4 μm) was formed on a PVC intermediate layer in the same manner as in Example 7, except that the hydrophilic polymer 1 and the polyurethane E2000 were mixed such that the mass ratio (solid content ratio) of the hydrophilic polymer 1/E2000 was changed to 100/800 as compared with that in Example 7. Accordingly, a wire (sample 12) in which the lubricating layer containing the hydrophilic polymer 1 and E2000 at a mixing ratio of 100/800 was formed on the PVC intermediate layer was obtained.

Example 13

A lubricating layer (dry film thickness: 4 μm) was formed on a PVC intermediate layer in the same manner as in Example 7, except that the hydrophilic polymer 1 and the polyurethane E2000 were mixed such that the mass ratio (solid content ratio) of the hydrophilic polymer 1/E2000 was changed to 100/1000 as compared with that in Example 7. Accordingly, a wire (sample 13) in which the lubricating layer containing the hydrophilic polymer 1 and E2000 at a mixing ratio of 100/1000 was formed on the PVC intermediate layer was obtained.

Example 14

A lubricating layer (dry film thickness: 4 μm) was formed on a PVC intermediate layer in the same manner as in Example 7, except that the hydrophilic polymer 1 and the polyurethane E2000 were mixed such that the mass ratio (solid content ratio) of the hydrophilic polymer 1/E2000 was changed to 100/1200 as compared with that in Example 7. Accordingly, a wire (sample 14) in which the lubricating layer containing the hydrophilic polymer 1 and E2000 at a mixing ratio of 100/1200 was formed on the PVC intermediate layer was obtained.

[Sliding Durability Evaluation 3]

Regarding the samples 7 to 14 in the above Examples 7 to 14, the sliding durability of the lubricating layer was evaluated in accordance with the method and the determination criteria described in the above [Sliding Durability Evaluation 2]. The results are shown in the following Table 3.

[Table 3]

| | Lubricating layer | Sliding durability |
|---|---|---|
| | Mixing ratio (mass ratio) of hydrophilic polymer 1/E2000 | |
| Example 8 | 100/50 | C |
| Example 9 | 100/100 | B |
| Example 10 | 100/200 | A |
| Example 7 | 100/400 | A |
| Example 11 | 100/600 | A |
| Example 12 | 100/800 | B |
| Example 13 | 100/1000 | B |
| Example 14 | 100/1200 | C |

Example 15

The hydrophilic polymer 1 was obtained in the same manner as in Example 4.

The hydrophilic polymer 1 and the polyurethane E2000 were mixed such that a mass ratio (solid content ratio) of the hydrophilic polymer 1/E2000 was 100/400. The mixture was adjusted such that a total solid content concentration of resin components (the hydrophilic polymer 1 and E2000) was 6.0 mass% and a mixing ratio (mass ratio) of acetone to water was 50/50, and a coating liquid was obtained.

A Ni-Ti wire (outer diameter = 0.34 mm) was coated with a thermoplastic polyurethane resin in a thickness of about 35 μm to obtain a coated wire (outer diameter = 0.41 mm) in which a polyurethane resin film (polyurethane underlayer) was formed on the wire.

Next, the coated wire was immersed in the coating liquid prepared as described above for 5 seconds, then pulled up at a velocity of 50 mm/sec, and then dried for 2 hours in a drying furnace maintained at 120°C, and a lubricating layer (dry film thickness: 4 μm) was formed on the coated wire. Accordingly, a wire (sample 17) in which the lubricating layer containing the hydrophilic polymers 1 and E2000 was formed on the polyurethane resin film was obtained.

[Sliding Durability Evaluation 4]

Regarding the samples 7 and 15 in the above Examples 7 and 15, the sliding durability of the lubricating layer was evaluated in accordance with the method and the determination criteria described in the above [Sliding Durability

Evaluation 2]. The results are shown in the following Table 4.

[Table 4]

| | Base layer | Sliding durability |
|---|---|---|
| Example 7 | Polyvinyl chloride (PVC) | A |
| Example 15 | Polyurethane resin | B |

Example 16

A coated wire 2 in which a polyurethane resin film (polyurethane underlayer) (thickness: about 35 μm) was formed on a wire was obtained in the same manner as in Example 5 except that a Ni-Ti wire having a shape shown in FIG. 3 was used instead of the Ni-Ti wire (outer diameter = 0.34 mm) in Example 5. Next, a PVC film (PVC intermediate layer) (thickness: about 4 μm) was formed on a surface of the coated wire 2 (base material wire 2) in the same manner as in Example 5 except that the coated wire 2 obtained as described above was used instead of the coated wire 1 in Example 5. That is, the base wire 2 has a structure in which the polyurethane resin film (polyurethane underlayer) (thickness: about 35 μm) and the PVC film (PVC intermediate layer) (thickness: about 4 μm) are sequentially formed on the wire surface.

As shown in FIG. 3, a Ni-Ti wire (total length: 1800 mm) 30 used in this example has a structure in which a region from a distal end 31 to 10 mm therefrom (distal end straight part 32) has a straight shape with a diameter of 0.085 mm, a region from 10 mm to 260 mm away from the distal end 31 (tapered part 33) has a tapered shape with a diameter increasing from 0.085 mm to 0.34 mm, and a region at a distance of 260 mm or more from the distal end has a straight shape with a diameter of 0.34 mm and continues to a rear end portion 34. In addition, as shown in FIG. 5B, a part (distal end straight part) from the distal end to 10 mm therefrom is subjected to a heat treatment so as to have a shape inclined by about 45°.

The coated wire 2 obtained by coating the Ni-Ti wire with a thermoplastic polyurethane resin in a thickness of about 35 μm has a structure in which the region from the distal end to 10 mm therefrom (distal end straight part) has a straight shape with a diameter of 0.155 mm, the region from 10 mm to 260 mm away from the distal end (tapered part) has a tapered shape with a diameter increasing from 0.155 mm to 0.41 mm, and the region at a distance of 260 mm or more from the distal end has a straight shape with a diameter of 0.41 mm and continues to the rear end. Similarly, the base material wire 2 obtained by coating the coated wire 2 with PVC in a thickness of about 4 μm has a structure in which the region from the distal end to 10 mm therefrom (distal end straight part) has a straight shape with a diameter of 0.163 mm, the region from 10 mm to 260 mm away from the distal end (tapered part) has a tapered shape with a diameter increasing from 0.163 mm to 0.418 mm, and the region at a distance of 260 mm or more from the distal end has a straight shape with a diameter of 0.418 mm and continues to the rear end.

A lubricating layer (dry film thickness: 4 μm) was formed on a PVC intermediate layer in the same manner as in Example 7 except that the above base material wire 2 was used instead of the base material wire 1 in Example 7. Accordingly, a wire (sample 16) in which the lubricating layer containing the hydrophilic polymer 1 and E2000 at a mixing ratio of 100/400 was formed on the PVC intermediate layer was obtained.

Comparative Example 10

A polyurethane resin film (polyurethane underlayer) (thickness: about 35 μm) was formed on a Ni-Ti wire having the shape shown in FIG. 3 in a method similar to the method in Example 16 (coated wire 2). Next, the coated wire 2 was immersed in a tetrahydrofuran (THF) solution containing polyvinyl chloride (PVC) and 4,4'-diphenylmethane diisocyanate (MDI) (PVC concentration = 5 mass%, MDI concentration = 5 mass%) for 5 seconds under an environment of 25°C and a relative humidity of 45% RH, then pulled up at a velocity of 50 mm/sec, and dried at the room temperature (25°C) for 30 minutes, and a film containing PVC and MDI (PVC/MDI intermediate layer) (thickness: about 4 μm) was formed on a surface of the coated wire 2 (base material wire 3).

The base material wire 3 was immersed for 5 seconds in a 1 mass% THF solution containing a half ethyl ester (degree of esterification: 40% to 50%) of a methyl vinyl ether maleic anhydride copolymer (GANTREZ AN-169, manufactured by ISP), then pulled up at a velocity of 50 mm/sec, and dried at about 60°C for 12 hours (base material wire 3'). Next, the base material wire 3' was immersed for 30 minutes in warm water (pH 8.2) at about 60°C in which 0.1 mass% of NaCl and 0.05 mass% of sodium hydrogen carbonate ($NaHCO_3$) were dissolved, then was washed with warm water at about 60°C for 1 minute, and dried at the room temperature (25°C), and a wire in which a lubricating layer (dry film thickness: about 1 μm to 2 μm) was formed on the PVC/MDI intermediate layer was obtained (comparative sample 10).

Comparative Example 11

A wire (comparative sample 11) in which a lubricating layer (dry film thickness: about 1 μm to 2 μm) was formed on a PVC intermediate layer was obtained in the same manner as in Comparative Example 10 except that 4,4'-diphenylmethane diisocyanate (MDI) in Comparative Example 10 was not used.

[Sliding Durability Evaluation 5]

Regarding the sample 16 in the above Example 16 and the comparative samples 10 and 11 in the above Comparative Examples 10 and 11, the sliding durability of the lubricating layer was evaluated in accordance with the method and the determination criteria described in the above [Sliding Durability Evaluation 2]. The results are shown in the following Table 5. In the Evaluation 5, the region at a distance of 260 mm or more from the distal end was brought into contact with the terminal (φ10 mm) 4 made of hydrogenated styrene-based thermoplastic elastomer (SEBS), and the sliding durability in the region at a distance of 260 mm or more from the distal end was evaluated.

[Distal End Abutting Resistance Evaluation]

Regarding the sample 16 in the above Example 16 and the comparative samples 10 and 11 in the above Comparative Examples 10 and 11, the distal end abutting resistance was evaluated using a micro-load meter 40 shown in FIG. 4. Regarding the samples in which a lubricating layer (dry film thickness: 4 μm) was formed on a PVC intermediate layer on the base material wire 2 obtained in the same manner as in Example 16 except that the coating liquids prepared in Examples 1 to 15 were used instead of that in Example 16, the distal end abutting resistance was evaluated as "A" in all cases according to the following method and determination criteria.

That is, a position of 10 mm away from the distal end of each sample 43 is fixed by a chuck 44 attached to a load cell 42, and the sample 43 is pushed down vertically at a test velocity of 5 mm/sec toward a pedestal 46 to which a #1200 abrasive paper (a slide stopper abrasive paper) (not shown) is attached. A load ($L_1$ (gf)) at a time point when the distal end of the sample is pushed down by 2 mm after the distal end comes into contact with the abrasive paper is measured. In addition, regarding the coated wire 2, a load ($L_0$ (gf)) at the time point when the distal end is pushed down by 2 mm is measured in the same manner as described above. Each measurement was performed three times, and an average value thereof was adopted.

Regarding each sample, a difference (= $L_1$ (gf) - $L_0$ (gf) (absolute value)) between the load ($L_0$ (gf)) on the coated wire 2 (polyurethane resin film) and the load ($L_1$ (gf)) on each sample (in which the PVC intermediate layer and the lubricating layer were laminated) was calculated. The results are shown in the following Table 5. In the following Table 5, a case where the above difference (= $L_1$ (gf) - $L_0$ (gf) (absolute value)) is less than 0.1 gf is indicated by "A", and a case where the difference is 0.1 gf or more is indicated by "B". A small difference (= $L_1$ (gf) - $L_0$ (gf) (absolute value)) indicates flexibility.

[Table 5]

| | Sliding durability | Distal end abutting resistance |
|---|---|---|
| Example 16 | A | A |
| Comparative Example 10 | A | B |
| Comparative Example 11 | D | A |

[Guide Wire Distal End Reachability Evaluation Using Blood Vessel Model]

Regarding the sample 16 in the above Example 16 and the comparative samples 10 and 11 in the above Comparative Examples 10 and 11, the guide wire distal end reachability was evaluated using a blood vessel model 50 shown in A of FIG. 5 in accordance with the following method. A of FIG. 5 is a plan view showing a part of the blood vessel model.

In the blood vessel model 50, a hollow passage was formed inside a plate-shaped member made of a transparent resin. The passage included a straight main pipe 51 and a side branch 52 branched from the main pipe 51. An inner diameter of the main pipe 51 was 3 mm. An inner diameter of the side branch 52 was 2.2 mm. The side branch 52 has six arc-shaped curved portions 53 alternately curved in opposite directions, and linear portions 54 connecting the adjacent curved portions 53. The curvature radius R of a center line passing through a center of an inner cavity of the curved portion 53 was 4.5 mm. All the straight portions 54 were parallel to each other and inclined at an inclination angle θ of 50° with respect to the main pipe 51. A length S of the straight portion 54 was 5 mm.

After the blood vessel model is filled with water, each sample is inserted into the side branch 52 from the main pipe

51, and then the sample is pushed forward while being appropriately rotated and adjusting a direction of the distal end so as to be easily advanced. Each sample was used in a shape in which a part from the distal end to 10 mm therefrom (distal end straight part) was inclined by about 45° as shown in B of FIG. 5 so that the distal end of the sample could be inserted into the side branch 52 from the main pipe 51.

As a result, a distal end of the sample 16 could be easily pushed forward through two or more curved portions 53 after being inserted into the side branch 52. From the above results, it is considered that the medical device according to the present invention can be easily inserted into a complicated lesion site. It is considered that the above results are obtained because the sample 16 is excellent in the sliding durability even under a very severe situation and is excellent in the flexibility (particularly, the flexibility of the distal part).

In contrast, a distal end of the comparative sample 10 could not exceed the second curved portion 53 after being inserted into the side branch 52. From the results in the above Table 5, it is considered that the above results are obtained because the comparative sample 10 is inferior in the flexibility (particularly, flexibility of the distal part).

In addition, a distal end of the comparative sample 11 could not be pushed forward immediately after being inserted into the side branch 52. It is presumed that this is because, as shown in the above Table 3, the comparative sample 11 is inferior in the sliding durability (the sliding resistance value increases even under a slight load).

Example 17

The hydrophilic polymer 1 was obtained in the same manner as in Example 1.

The hydrophilic polymer 1 obtained as described above, the polyurethane E2000, and a reactive aqueous polyisocyanate (Elastron (registered trademark) BN-77, manufactured by DAIICHI KOGYO SEIYAKU CO., LTD.; hereinafter, also referred to as "Elastron BN-77" or "BN-77") were mixed such that a mass ratio (solid content ratio) of the hydrophilic polymer 1/E2000/BN-77 was 100/400/5. The mixture was adjusted such that a total solid content concentration of resin components (the hydrophilic polymer 1, E2000, and BN-77) was 6.2 mass% and a mixing ratio (mass ratio) of acetone to water was 50/50, followed by stirring for 1 hour, and a coating liquid was obtained.

A lubricating layer (dry film thickness: 4 μm) was formed on a PVC intermediate layer in the same manner as in Example 16 except that the coating liquid prepared as described above was used instead of that in Example 16. Accordingly, a wire (sample 17) in which the lubricating layer containing the hydrophilic polymer 1, E2000, and BN-77 at a mixing ratio of 100/400/5 was formed on the PVC intermediate layer was obtained.

Example 18

The hydrophilic polymer 1 was obtained in the same manner as in Example 1.

The hydrophilic polymer 1 obtained as described above, the polyurethane E2000, and the Elastron BN-77 were mixed such that a mass ratio (solid content ratio) of the hydrophilic polymer 1/E2000/BN-77 was 100/400/20. The mixture was adjusted such that a total solid content concentration of resin components (the hydrophilic polymer 1, E2000, and BN-77) was 6.2 mass% and a mixing ratio (mass ratio) of acetone to water was 50/50, followed by stirring for 1 hour, and a coating liquid was obtained.

A lubricating layer (dry film thickness: 4 μm) was formed on a PVC intermediate layer in the same manner as in Example 16 except that the coating liquid prepared as described above was used instead of that in Example 16. Accordingly, a wire (sample 18) in which the lubricating layer containing the hydrophilic polymer 1, E2000, and BN-77 at a mixing ratio of 100/400/20 was formed on the PVC intermediate layer was obtained.

Example 19

The hydrophilic polymer 1 was obtained in the same manner as in Example 1.

The hydrophilic polymer 1 obtained as described above, the polyurethane E2000, and the Elastron BN-77 were mixed such that a mass ratio (solid content ratio) of the hydrophilic polymer 1/E2000/BN-77 was 100/400/40. The mixture was adjusted such that a total solid content concentration of resin components (the hydrophilic polymer 1, E2000, and BN-77) was 6.2 mass% and a mixing ratio (mass ratio) of acetone to water was 50/50, followed by stirring for 1 hour, and a coating liquid was obtained.

A lubricating layer (dry film thickness: 4 μm) was formed on a PVC intermediate layer in the same manner as in Example 16 except that the coating liquid prepared as described above was used instead of that in Example 16. Accordingly, a wire (sample 19) in which the lubricating layer containing the hydrophilic polymer 1, E2000, and BN-77 at a mixing ratio of 100/400/40 was formed on the PVC intermediate layer was obtained.

Example 20

The hydrophilic polymer 1 was obtained in the same manner as in Example 1.

The hydrophilic polymer 1 obtained as described above, the polyurethane E2000, and the Elastron BN-77 were mixed such that a mass ratio (solid content ratio) of the hydrophilic polymer 1/E2000/BN-77 was 100/400/60. The mixture was adjusted such that a total solid content concentration of resin components (the hydrophilic polymer 1, E2000, and BN-77) was 6.2 mass% and a mixing ratio (mass ratio) of acetone to water was 50/50, followed by stirring for 1 hour, and a coating liquid was obtained.

A lubricating layer (dry film thickness: 4 μm) was formed on a PVC intermediate layer in the same manner as in Example 16 except that the coating liquid prepared as described above was used instead of that in Example 16. Accordingly, a wire (sample 20) in which the lubricating layer containing the hydrophilic polymer 1, E2000, and BN-77 at a mixing ratio of 100/400/60 was formed on the PVC intermediate layer was obtained.

Example 21

The hydrophilic polymer 1 was obtained in the same manner as in Example 1.

The hydrophilic polymer 1 obtained as described above, the polyurethane E2000, and the Elastron BN-77 were mixed such that a mass ratio (solid content ratio) of the hydrophilic polymer 1/E2000/BN-77 was 100/400/80. The mixture was adjusted such that a total solid content concentration of resin components (the hydrophilic polymer 1, E2000, and BN-77) was 6.2 mass% and a mixing ratio (mass ratio) of acetone to water was 50/50, followed by stirring for 1 hour, and a coating liquid was obtained.

A lubricating layer (dry film thickness: 4 μm) was formed on a PVC intermediate layer in the same manner as in Example 16 except that the coating liquid prepared as described above was used instead of that in Example 16. Accordingly, a wire (sample 21) in which the lubricating layer containing the hydrophilic polymer 1, E2000, and BN-77 at a mixing ratio of 100/400/80 was formed on the PVC intermediate layer was obtained.

Regarding the samples 17 to 21 in the above Examples 17 to 21, the sliding durability of the lubricating layer was evaluated in accordance with the method and the determination criteria described in the above [Sliding Durability Evaluation 2]. In addition, regarding the samples 17 to 21 in the above Examples 17 to 21, the distal end abutting resistance was evaluated in accordance with the method and the determination criteria described in the above [Distal End Abutting Resistance Evaluation]. The results are shown in the following Table 6.

[Microparticle Test]

In order to study the effect of the addition of the polyisocyanate, the above samples 16 to 21 in Examples 16 to 21 were evaluated for the generation of microparticles under a more severe situation using a test system shown in FIG. 6 in accordance with the following method.

A test system 60 shown in FIG. 6 includes a mold 63 having a total length of 450 mm and an inner diameter of 3 mm, which is bent by providing three bent portions in a wavy line shape at an angle of about 80° with a curvature radius of 15 mm. A 4 Fr catheter tube 62 of 100 cm or more is set in the mold 63. Distilled water (100 mL) is injected from a hub 61 of the catheter, and a liquid coming out of a distal end of the catheter is collected and used as a blank liquid.

Next, each sample is set in the catheter tube 62 filled with distilled water, and is reciprocated (slid) ten times with a sliding distance (stroke) of 100 cm. After sliding is performed for predetermined number of times, the sample is taken out from the catheter tube 62, 100 mL of distilled water is injected into the catheter tube 62, and a test fluid discharged from the distal end of the catheter is collected.

The number of microparticles having a size (diameter) of 10 μm or more in the test liquid and the blank liquid (each 100 mL) collected as described above was measured by a liquid particle measuring instrument (device name: light shielding type automatic particle measuring device, manufactured by HIAC Corporation), and a value (= $Y_1$ - $Y_0$) obtained by subtracting the measured value ($Y_0$) of the number of microparticles in the blank liquid from the number ($Y_1$) of microparticles in the test liquid was defined as the number of microparticles in the test sample.

As a result, the numbers of microparticles in the samples 16 to 21 were sufficiently low in both the blank liquid and the test liquid. In addition, a proportion of the number of microparticles in each of the samples 17 to 21 to the number of microparticles in the sample 16 (= number of microparticles in each sample/number of microparticles in sample 16) was calculated, and the results ("ratio of the number of microparticles" in the following table) are shown in the following Table 6.

[Table 6]

| | Mixing mass ratio of hydrophilic polymer 1/E2000/BN-77 | Sliding durability | Distal end abutting resistance | Ratio of number of microparticles |
|---|---|---|---|---|
| Example 16 | 100/400/0 | A | A | - |
| Example 17 | 100/400/5 | A | A | 1/2 |
| Example 18 | 100/400/20 | A | A | 1/3 |
| Example 19 | 100/400/40 | A | A | 1/3 |
| Example 20 | 100/400/60 | A | A | 1/3 |
| Example 21 | 100/400/80 | B | A | 1/3 |

From the results in Table 6, it is understood that the number of microparticles is reduced by further adding the polyisocyanate. It is presumed that the above results are caused by the increased film strength of the surface lubricating layer. In addition, from the above results, it is considered that the use of the polyisocyanate can exhibit an excellent lubricating property and lubrication retaining property (sliding durability) even in a more severe situation.

The present application is based on Japanese patent application No. 2021-107517 filed on June 29, 2021, the entire content of which is incorporated herein by reference.

Reference Signs List

1 water
2 petri dish
3 sample (medical device)
4 terminal
5 load
6 moving table
10 friction tester
30 Ni-Ti wire
31 distal end
32 distal end straight part
33 tapered part
34 rear end portion
40 micro-load meter
42 load cell
43 sample
44 sample chuck portion
46 base
51 main pipe
52 side branch
53 curved portion
54 linear portion
50 blood vessel model
61 hub
62 catheter tube
63 mold
60 test system
101 base layer
101a base layer core portion
101b base material surface layer
102 surface lubricating layer
100 medical device

## Claims

1. A medical device comprising:

   a base layer; and
   a surface lubricating layer formed on at least a part of the base layer, wherein
   the surface lubricating layer contains a polymer having a carboxy group, or a salt or an ester thereof, and a polyurethane, and
   the polyurethane has an elongation percentage of 700% or more and a Young's modulus of 5.0 $N/mm^2$ or more.

2. The medical device according to claim 1, wherein the polyurethane is contained in the surface lubricating layer in a proportion of 50 parts by mass to 1200 parts by mass based on 100 parts by mass of the polymer.

3. The medical device according to claim 2, wherein the polyurethane is contained in the surface lubricating layer in a proportion of 100 parts by mass to 1000 parts by mass based on 100 parts by mass of the polymer.

4. The medical device according to any one of claims 1 to 3, wherein the polymer is at least one type selected from the group consisting of hyaluronic acid or an alkali metal salt or a Group 2 metal salt thereof, polyacrylic acid or an alkali metal salt or a Group 2 metal salt thereof, and a polymer having a structural unit represented by the following formula (1):

[Chem. 1]

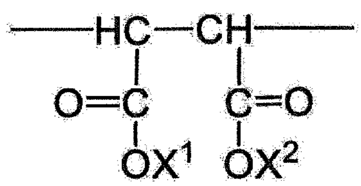
(1)

wherein $X^1$ and $X^2$ each independently represent a hydrogen atom, an alkali metal, a Group 2 metal, or a linear or branched alkyl group having 1 to 24 carbon atoms.

5. The medical device according to any one of claims 1 to 3, wherein the surface lubricating layer further contains a polyisocyanate or a crosslinked product thereof.

6. The medical device according to any one of claims 1 to 3, which is a catheter or a guide wire.

7. A method for producing a medical device, the method comprising: preparing or selecting a polyurethane having an elongation percentage of 700% or more and a Young's modulus of 5.0 $N/mm^2$ or more;

   preparing a coating liquid containing the polyurethane and a polymer having a carboxy group, or a salt or an ester thereof; and
   coating a base layer with the coating liquid to form a surface lubricating layer on the base layer.

## Patentansprüche

1. Medizinische Vorrichtung, umfassend:

   eine Grundschicht; und
   eine Oberflächenschmierschicht, die auf mindestens einem Teil der Grundschicht ausgebildet ist, wobei
   die Oberflächenschmierschicht ein Polymer mit einer Carboxygruppe oder ein Salz oder einen Ester davon und ein Polyurethan enthält, und
   das Polyurethan eine Dehnung von 700 % oder mehr und einen Elastizitätsmodul von 5,0 $N/mm^2$ oder mehr aufweist.

2. Medizinische Vorrichtung nach Anspruch 1, wobei das Polyurethan in der Oberflächenschmierschicht in einem Anteil

von 50 Masseteilen bis 1200 Masseteilen, bezogen auf 100 Masseteile des Polymers, enthalten ist.

3. Medizinische Vorrichtung nach Anspruch 2, wobei das Polyurethan in der Oberflächenschmierschicht in einem Anteil von 100 Masseteilen bis 1000 Masseteilen, bezogen auf 100 Masseteile des Polymers, enthalten ist.

4. Medizinische Vorrichtung nach einem der Ansprüche 1 bis 3, wobei das Polymer mindestens einen Typ umfasst, der aus der Gruppe ausgewählt ist, die aus Hyaluronsäure oder einem Alkalimetallsalz oder einem Metallsalz der Gruppe 2 davon, Polyacrylsäure oder einem Alkalimetallsalz oder einem Metallsalz der Gruppe 2 davon und einem Polymer mit einer Struktureinheit, die durch die folgende Formel (1) dargestellt wird, besteht:

[Chem. 1]

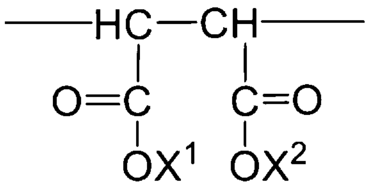

(1)

wobei $X^1$ und $X^2$ jeweils unabhängig voneinander ein Wasserstoffatom, ein Alkalimetall, ein Metall der Gruppe 2 oder eine lineare oder verzweigte Alkylgruppe mit 1 bis 24 Kohlenstoffatomen darstellen.

5. Medizinische Vorrichtung nach einem der Ansprüche 1 bis 3, wobei die Oberflächenschmierschicht ferner ein Polyisocyanat oder ein vernetztes Produkt davon enthält.

6. Medizinische Vorrichtung nach einem der Ansprüche 1 bis 3, die ein Katheter oder ein Führungsdraht ist.

7. Verfahren zur Herstellung einer medizinischen Vorrichtung, wobei das Verfahren umfasst: Herstellen oder Auswählen eines Polyurethans mit einer Dehnung von 700 % oder mehr und einem Elastizitätsmodul von 5,0 $N/mm^2$ oder mehr;

Herstellen einer Beschichtungsflüssigkeit, die das Polyurethan und ein Polymer mit einer Carboxygruppe oder ein Salz oder einen Ester davon enthält; und
Beschichten einer Grundschicht mit der Beschichtungsflüssigkeit, um eine Oberflächenschmierschicht auf der Grundschicht zu bilden.

## Revendications

1. Dispositif médical comprenant :

une couche de base ; et
une couche lubrifiante de surface formée sur au moins une partie de la couche de base, dans lequel
la couche lubrifiante de surface contient un polymère ayant un groupe carboxy, ou un sel ou un ester de celui-ci, et un polyuréthane, et
le polyuréthane a un pourcentage d'allongement de 700 % ou plus et un module de Young de 5,0 $N/mm^2$ ou plus.

2. Dispositif médical selon la revendication 1, dans lequel le polyuréthane est contenu dans la couche lubrifiante de surface dans une proportion de 50 parties en masse à 1200 parties en masse sur la base de 100 parties en masse du polymère.

3. Dispositif médical selon la revendication 2, dans lequel le polyuréthane est contenu dans la couche lubrifiante de surface dans une proportion de 100 parties en masse à 1000 parties en masse sur la base de 100 parties en masse du polymère.

4. Dispositif médical selon l'une quelconque des revendications 1 à 3, dans lequel le polymère est au moins un type sélectionné dans le groupe constitué par l'acide hyaluronique ou un sel de métal alcalin ou un sel de métal du groupe 2 de celui-ci, un acide polyacrylique ou un sel de métal alcalin ou un sel de métal du groupe 2 de celui-ci, et un polymère

ayant un motif structurel représenté par la formule (1) suivante :

[Chem. 1]

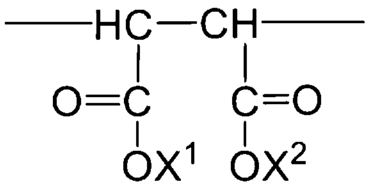

(1)

dans laquelle $X^1$ et $X^2$ représentent chacun indépendamment un atome d'hydrogène, un métal alcalin, un métal du groupe 2 ou un groupe alkyle linéaire ou ramifié ayant 1 à 24 atomes de carbone.

**5.** Dispositif médical selon l'une quelconque des revendications 1 à 3, dans lequel la couche lubrifiante de surface contient en outre un polyisocyanate ou un produit réticulé de celui-ci.

**6.** Dispositif médical selon l'une quelconque des revendications 1 à 3, qui est un cathéter ou un fil guide.

**7.** Procédé de fabrication d'un dispositif médical, le procédé comprenant : la préparation ou la sélection d'un polyuréthane ayant un pourcentage d'allongement de 700 % ou plus et un module de Young de 5,0 $N/mm^2$ ou plus ;

la préparation d'un liquide de revêtement contenant le polyuréthane et un polymère ayant un groupe carboxy, ou un sel ou un ester de celui-ci ; et

le revêtement d'une couche de base avec le liquide de revêtement pour former une couche lubrifiante de surface sur la couche de base.

[FIG. 1]

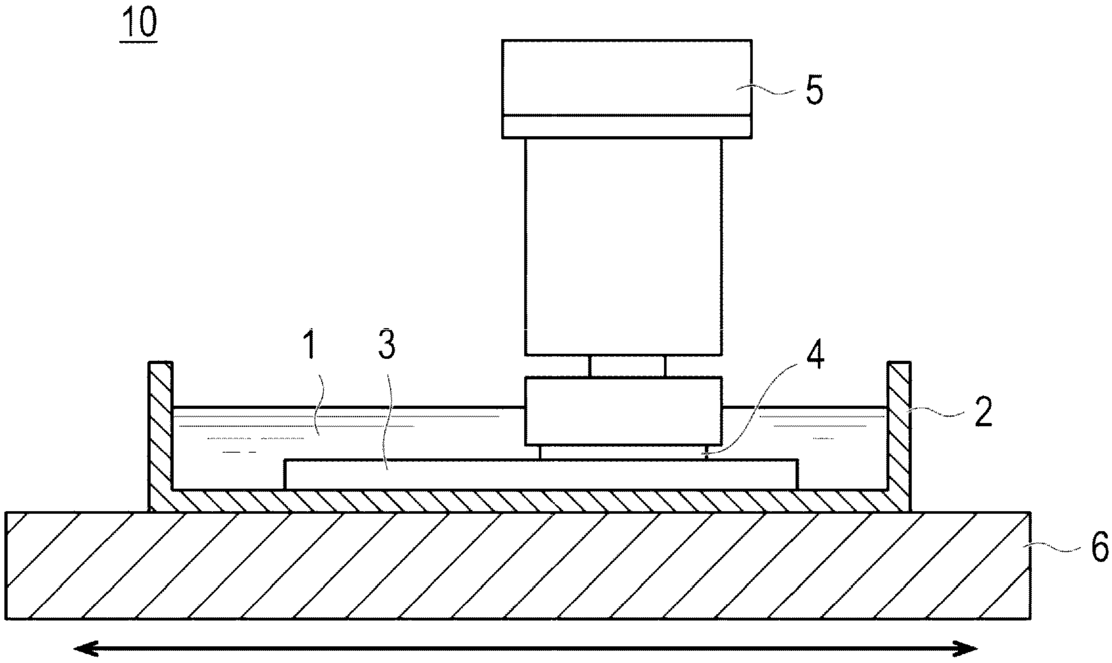
10
5
1
3
4
2
6

[FIG. 2]

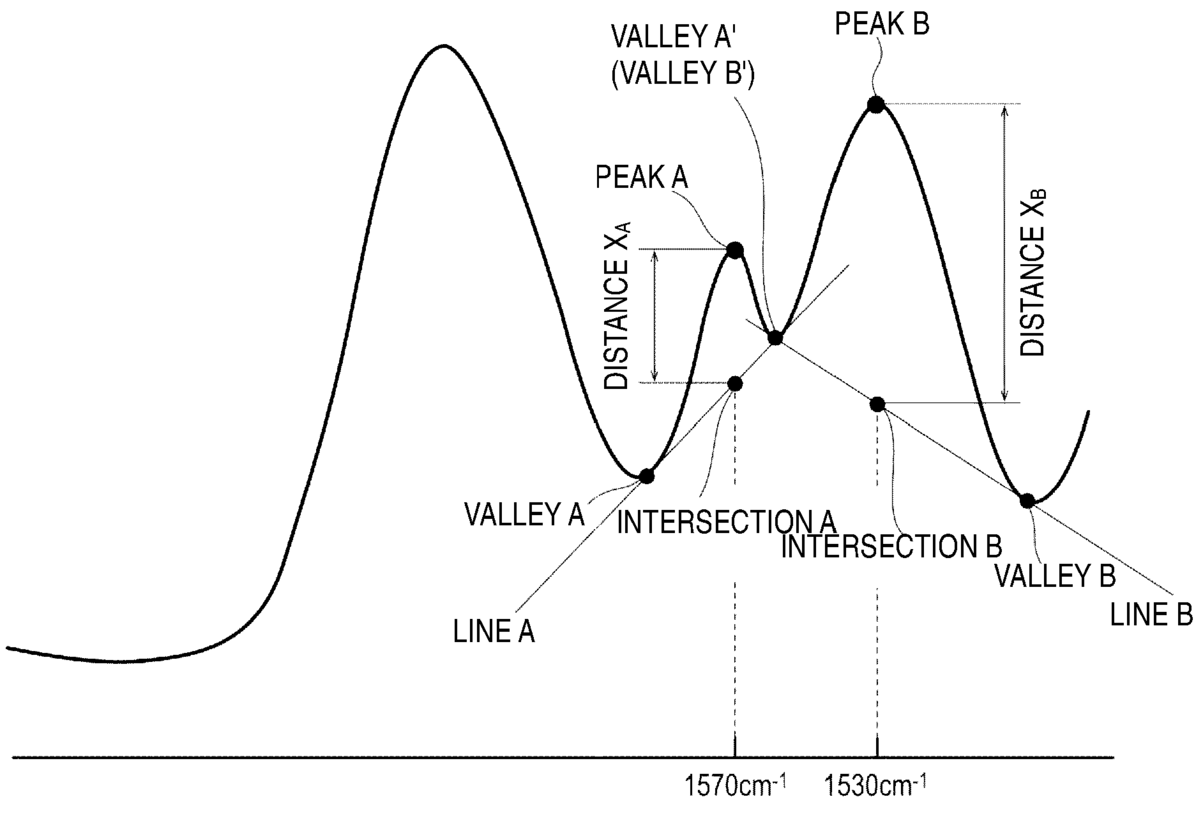
VALLEY A'
(VALLEY B')
PEAK B
PEAK A
DISTANCE $X_A$
DISTANCE $X_B$
VALLEY A
INTERSECTION A
INTERSECTION B
VALLEY B
LINE A
LINE B
1570cm$^{-1}$
1530cm$^{-1}$

[FIG. 3]

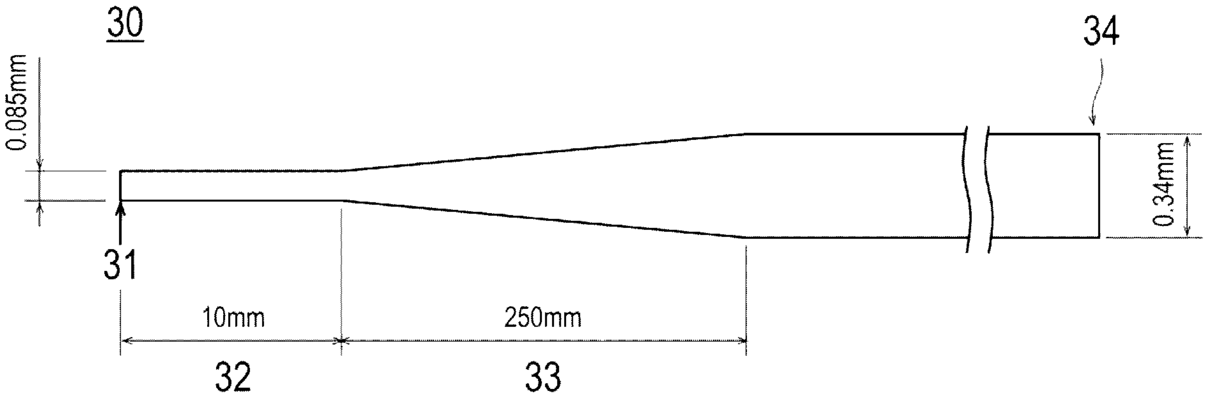
30
34
0.085mm
0.34mm
31
10mm
250mm
32
33

[FIG. 4]

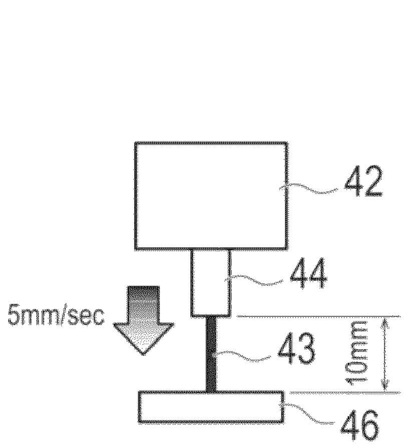
40
42
44
5mm/sec
43
10mm
46

[FIG. 5]

A

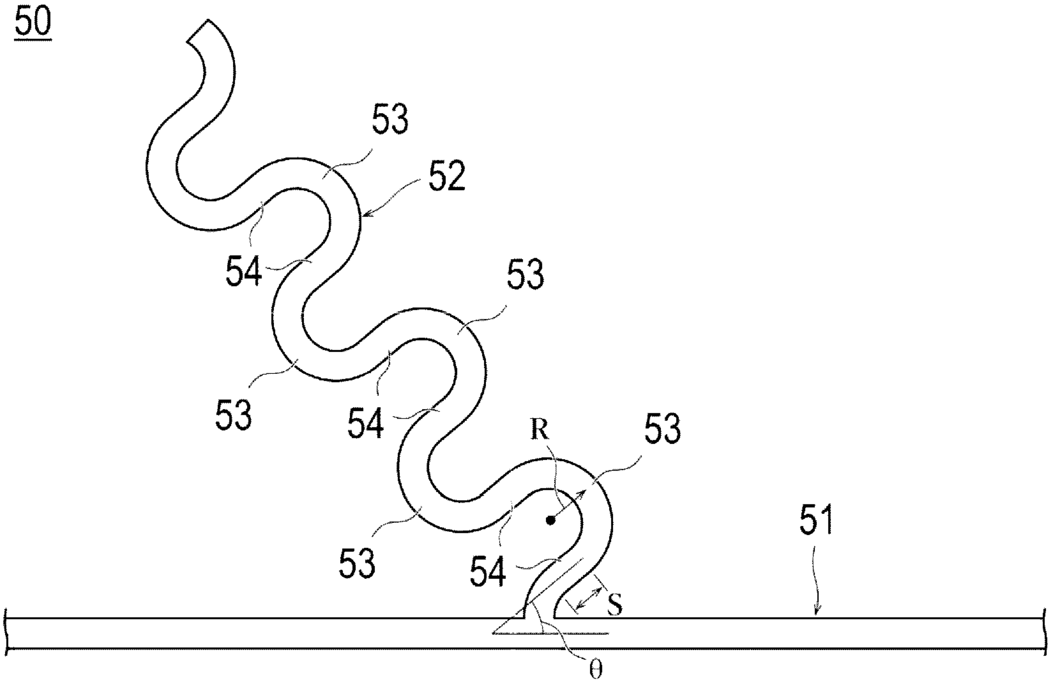


B

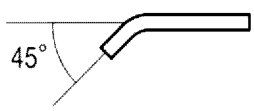


[FIG. 6]

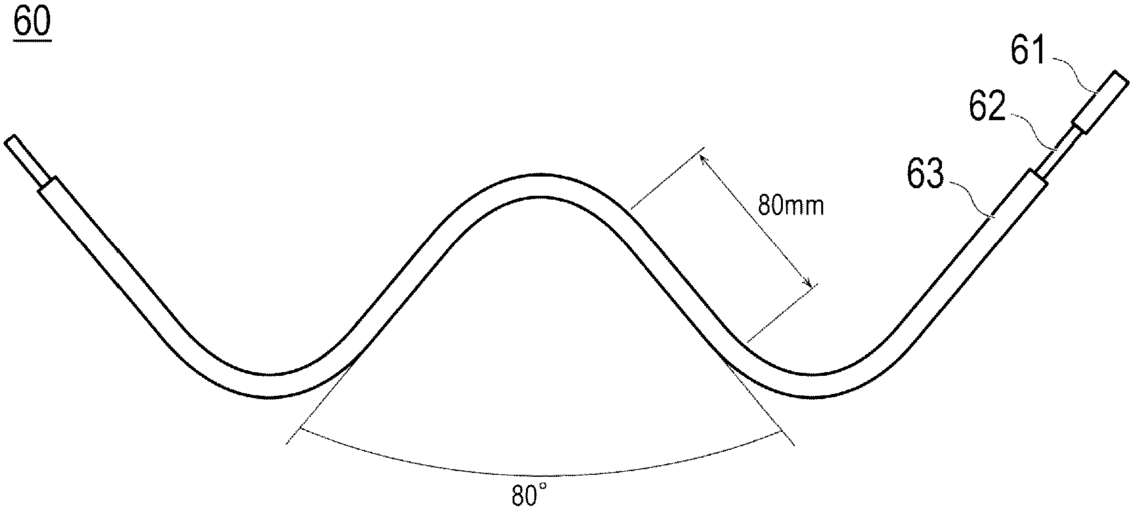

[FIG. 7]

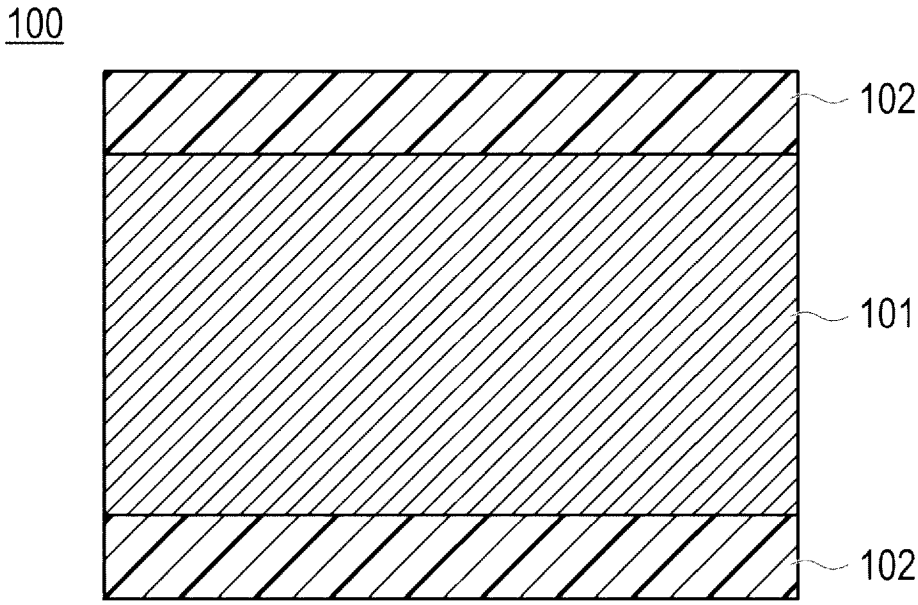


[FIG. 8]

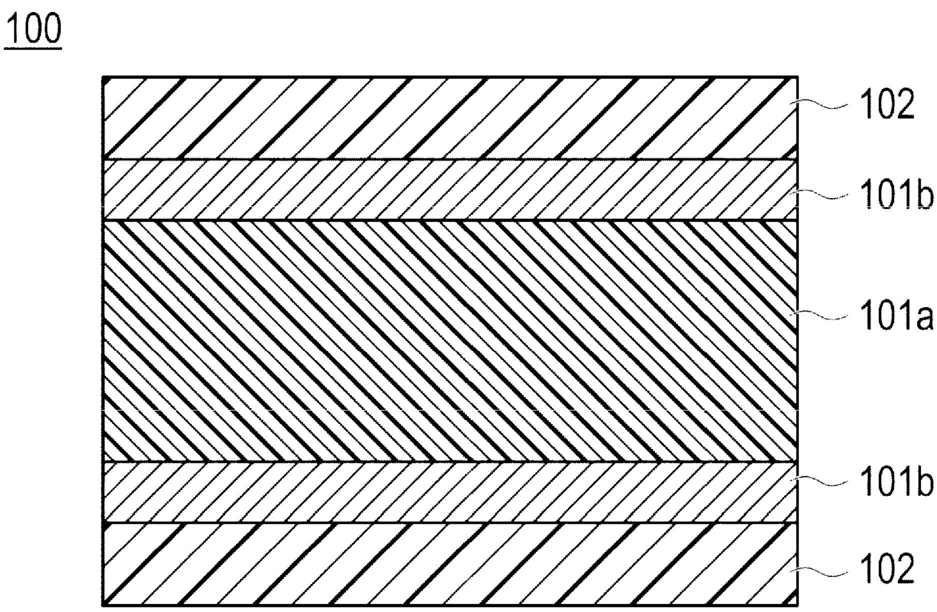

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015029625 A
- JP 2021107517 A